# EUROPEAN PATENT SPECIFICATION

(11) **EP 4 100 416 B1**
(45) Date of publication and mention of the grant of the patent: **16.07.2025**
(21) Application number: 21750997.5
(22) Date of filing: 08.02.2021
(51) Int. Cl.: C07H 21/02, C12N 15/11, C12Q 1/68, C12Q 1/6806, C12Q 1/6855, C12Q 1/686, C12Q 1/6869, C12N 15/10

(54) **COMPOSITIONS AND METHODS FOR RAPID RNA-ADENYLATION AND RNA SEQUENCING**
ZUSAMMENSETZUNGEN UND VERFAHREN ZUR SCHNELLEN RNA-ADENYLIERUNG UND RNA-SEQUENZIERUNG
COMPOSITIONS ET PROCÉDÉS POUR L'ADÉNYLATION ET LE SÉQUENÇAGE RAPIDE D'ARN

(30) Priority: 06.02.2020 US 202062971214 P
(43) Date of publication of application: 14.12.2022
(73) Proprietor: Cornell University, Ithaca, NY 14850 (US)
(72) Inventor: QIAN, Shu-Bing, Ithaca, NY 14850 (US); DONG, Leiming, Ithaca, NY 14850 (US); SHU, Xin, Ithaca, NY 14850 (US)
(74) Representative: FRKelly
(86) International application number: PCT/US2021/017110
(87) International publication number: WO 2021/159090

(56) References cited:
- EP-A1- 3 848 473
- WO-A1-2016/149021
- WO-A1-2019/099208
- WO-A1-2020/068302
- WO-A1-97/45535
- US-A1- 2006 051 801
- US-A1- 2015 159 202
- CHAKRAVARTY ANUPAM K. ET AL: "RNA 3'-Phosphate Cyclase (RtcA) Catalyzes Ligase-like Adenylylation of DNA and RNA 5'-Monophosphate Ends", JOURNAL OF BIOLOGICAL CHEMISTRY, vol. 286, no. 6, 22 November 2010 (2010-11-22), US, pages 4117 - 4122, XP093129789, ISSN: 0021-9258, Retrieved from the Internet <URL:https://www.ncbi.nlm.nih.gov/pmc/articles/PMC3039336/pdf/zbc4117.pdf> DOI: 10.1074/jbc.M110.196766
- D. B. MUNAFO ET AL: "Optimization of enzymatic reaction conditions for generating representative pools of cDNA from small RNA", RNA, vol. 16, no. 12, 4 October 2010 (2010-10-04), US, pages 2537 - 2552, XP055450328, ISSN: 1355-8382, DOI: 10.1261/rna.2242610
- SHIGEMATSU MEGUMI, KAWAMURA TAKUYA, KIRINO YOHEI: "Generation of 2′,3′-Cyclic Phosphate-Containing RNAs as a Hidden Layer of the Transcriptome", FRONTIERS IN GENETICS, vol. 9, 11 July 2018 (2018-07-11), pages 1 - 13, XP055848805
- ANUPAM K CHAKRAVARTY, STEWART SHUMAN: "RNA 3'-phosphate cyclase (RtcA) catalyzes ligase-like adenylylation of DNA and RNA 5'-monophosphate ends", J BIOL CHEM, vol. 286, no. 6, 22 November 2010 (2010-11-22), pages 4117 - 4122, XP055848808
- HORNSTEIN NICHOLAS, TORRES DANIELA, DAS SHARMA SOHANI, TANG GUOMEI, CANOLL PETER, SIMS PETER A.: "Ligation-free ribosome profiling of cell type-specific translation in the brain", GENOME BIOLOGY, vol. 17, 5 July 2016 (2016-07-05), pages 1 - 15, XP055848811
- LAMA ET AL.: "Adenylylation of small RNA sequencing adapters using the TS2126 RNA ligase I", RNA, vol. 22, 13 November 2015 (2015-11-13), pages 155 - 161, XP055543283, DOI: 10.1261/rna.054999.115

## Description

### FIELD

The present disclosure relates to improved compositions and methods for RNA sequencing.

### BACKGROUND

The emergence of genome-wide analysis to interrogate cellular DNA, RNA, and protein content has revolutionized the study of gene expression that mediates cellular homeostasis. RNA-seq uses next-generation sequencing (NGS) to reveal the presence and quantity of RNA molecules in a biological sample. Although RNA-seq is a powerful approach for quantifying gene expression, it cannot reliably identify previously unknown transcripts, splicing isoforms, and naturally occurred RNA fragments. Specialized RNA-seq methods have been designed to identify the 5 ends of transcripts (1), such as CAGE (cap analysis of gene expression), STRT (single-cell tagged reverse transcription), NanoCAGE (nano-cap analysis of gene expression), TSS-seq (oligo-capping), and GRO-cap (global nuclear run-on cap). However, these methods detect the presence of 5' end cap on RNA molecules. As a result, these approaches are not suitable for identification of RNA fragments, which are critical for studies of RNA metabolism. As one example, ribosome profiling (Ribo-seq) captures the entire set of ribosome-protected mRNA fragments (RPFs) generated by nuclease digestion followed by deep sequencing (2). It provides a snapshot of ribosome positions and density across the transcriptome at a sub-codon resolution. Since the commonly used RNA-seq approach cannot be applied to ribosome profiling, the broad application of Ribo-seq has been slowed by the complexity and expense of the protocol. Additionally, concerns have swirled around the interpretation of Ribo-seq results as details of sample preparation may introduce bias and artifacts (3). During library preparation, for instance, the efficiency of circularization or linker ligation could be influenced by the 5' end nucleotide identity of RPFs. As a result, technically inflated or depleted RPFs could alter the overall pattern of ribosome footprints. Additionally, pre-treatment with the translation inhibitor cycloheximide (CHX) has been shown to skew codon densities and induce unwanted cellular responses (4). Although omitting CHX pretreatment has become a common practice, eliminating artifacts introduced by varied protocols remains challenging.

A ligation-free Ribo-seq approach was recently introduced (5). However, the method relies on template-switching technology that is severely biased with a higher efficiency for RNA molecules having a G nucleotide in their 5' end (6). As a result, this ligation-free approach is not suitable for Ribo-seq due to the unacceptable 5' end bias that will distort the subsequent data analysis. For a similar reason, the ILLUMINA Ultra Low RNA sequencing kit (CLONTECH) cannot be used for Ribo-seq because it uses SMART (switching mechanism at the 5' end of the RNA transcript) to generate full-length cDNA copies of mRNA molecules. Although SMART method is capable of preparing cDNA for sequencing from single-cell amounts of RNA, it is time consuming, expensive and restricted to mRNA sequencing. For RNA fragments, SMART approach cannot faithfully capture RNA molecules with random 5' nucleotides. Challenges in RNA sequencing that are evident in previous approaches to Ribo-seq are also relevant to sequencing any RNA polynucleotides. Thus, there is an ongoing and unmet need for improved compositions and methods for use in sequencing RNA polynucleotides. The present disclosure is pertinent to this need.

WO2016/149021A1 discloses methods, compositions and kits for capturing, detecting and quantifying mature small RNAs. Embodiments of the methods comprise tailing both the 5' and 3' ends of mature small RNA by ligating a 5' ligation adaptor to the 5' end and polyadenylating the 3' end. Other embodiments comprise reverse transcribing the adaptor ligated, polyadenylated mature small RNA with a universal reverse transcription primer and amplifying the cDNA with universal primers.

WO2019099208A1 discloses a method for making a cDNA library comprising producing DNA:RNA hybrids, e.g., by reverse transcribing an RNA sample or by hybridizing DNA oligonucleotides to an RNA sample, treating the DNA:RNA hybrids with RNAseH to produce a digested sample that comprises fragments of the RNA; and (c) reverse transcribing the RNA fragments. Munafo et al 2010 (RNA, vol. 16, no. 12, pages 2537-2552) discloses enzyme compositions for use in RNA sequencing.

### SUMMARY

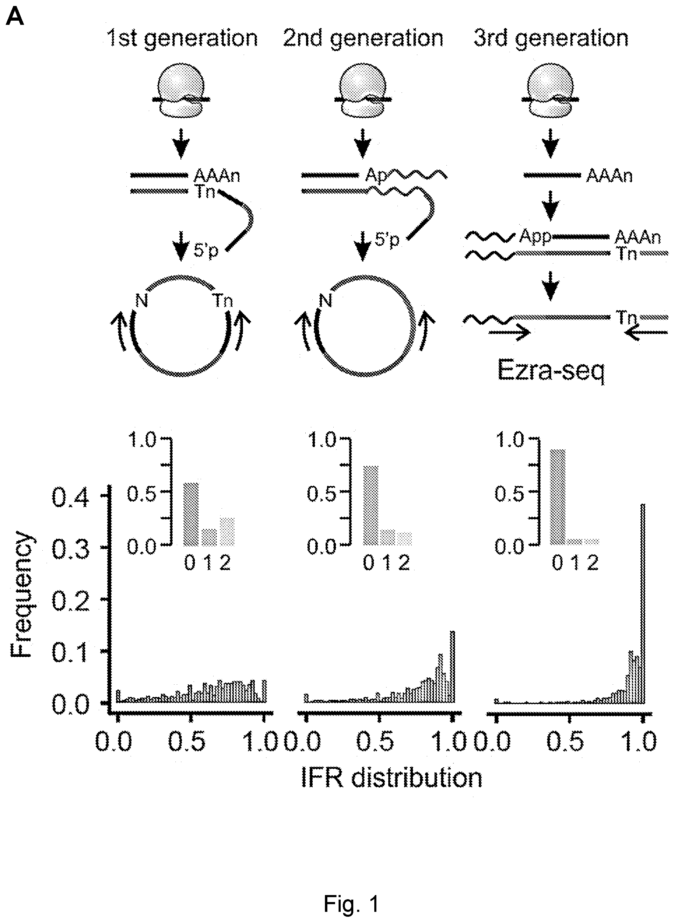

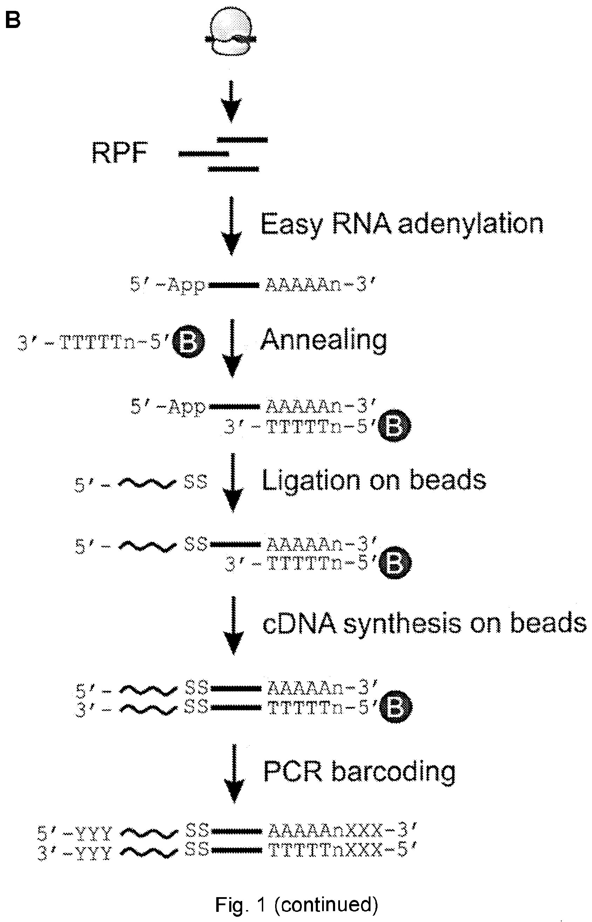

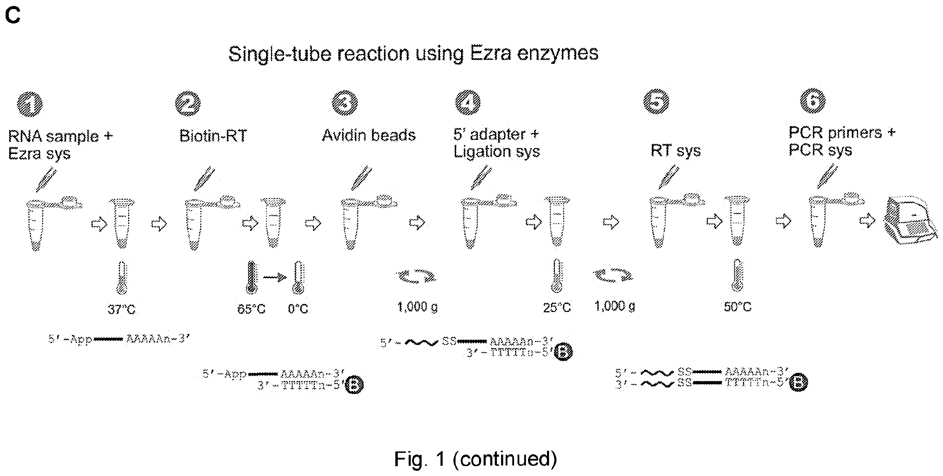

The present disclosure provides compositions and methods for use in RNA sequencing. The approach is referred to herein as **e**a**sy R**NA-**a**denylation **seq**uencing ("Ezra-seq"). A comparison of available methods to Ezra-seq in terms of sequencing ribosome protected fragments in provided in Figure 1A. An overview of the method is provided in Figure 1B. The disclosure provides for processing RNA samples and cDNA generation in a single tube, as generally depicted in Figure 1C.

The method for determining nucleotide sequences of RNA polynucleotides comprises:
a) providing a plurality of RNA fragments obtained from the RNA polynucleotides;
b) enzymatically phosphorylating 5' ends of the plurality of RNA fragments to provide a plurality of RNA fragments comprising mono-phosphorylated 5' ends;
c) enzymatically dephosphorylating 3' ends of the plurality of RNA fragments to provide a plurality of RNA fragments comprising free 3' hydroxyls;
   wherein a ligase enzymatically phosphorylates the 5' ends of the plurality of RNA fragments to provide a plurality of RNA fragments comprising mono-phosphorylated 5' ends, and wherein the ligase enzymatically dephosphorylates 3' ends of the plurality of RNA fragments to provide the plurality of RNA fragments comprising the free 3' hydroxyls;
d) enzymatically adenylylating phosphorylated 5' ends of the plurality of RNA fragments to provide a plurality of 5' mono-adenylated RNA fragments;
e) enzymatically polyadenylating 3' ends of the plurality of RNA fragments comprising the free 3' hydroxyls to provide a plurality of RNA fragments comprising polyadenylated 3' ends;
   wherein the enzymatically adenylating the phosphorylated 5' ends of the plurality of RNA fragments, and the polyadenylating of the 3' ends of the plurality of RNA fragments, is performed using a cyclase and a polymerase, wherein the cyclase comprises an RtcA enzyme;
f) ligating oligonucleotide adapters to the 5' ends of the plurality of 5' mono-adenylated RNA fragments, the oligonucleotide adapters optionally comprising a DNA/RNA hybrid 3' end, the DNA/RNA hybrid 3' end optionally comprising rCrC-OH or rGrG-OH as the RNA component of the DNA/RNA hybrid, to provide a plurality of RNA fragments comprising the oligonucleotide adapters at the 5' ends;
g) generating cDNAs from the plurality of RNA fragments of f);
h) amplifying the cDNAs; and
i) determining nucleotide sequences of the cDNAs, thereby determining the nucleotide sequences of the RNA polynucleotides.

The method comprises modification of RNA using mixtures of enzymes to produce cDNAs for sequencing, and further provides fusion proteins comprising segments of enzymes that can be used in the described method. The mixture of enzymes, contains, among other enzymes, a cyclase and a polymerase. The cyclase and a polymerase can be provided as a fusion protein.

In certain approaches, at least the described steps b)-h) are performed in a single reaction container. In certain approaches, the reaction container comprises a substrate, such as streptavidin. This may be used, for example, with a primer that is used to generate cDNAs by reverse transcription, the primer comprising a binding partner that binds to the substrate, for example a biotin moiety. The disclosure provides for improved 5' end sequencing.

The method is performed in part using a ligase to enzymatically phosphorylate 5' ends of RNA fragments to provide a plurality of RNA fragments comprising mono-phosphorylated 5' ends. The ligase also enzymatically dephosphorylates 3' ends of the RNA fragments to provide a plurality of RNA fragments comprising the free 3' hydroxyls. In certain approaches, the RNA polynucleotides modified by the ligase are further modified using the above-described cyclase and polymerase, which may be provided as separate proteins, or as components of a single fusion protein. Use of this approach facilitates enzymatically adenylating the phosphorylated 5' ends of the plurality of RNA fragments, and polyadenylating of the 3' ends of the plurality of RNA fragments. In non-limiting embodiments, polymerase comprises poly(A) polymerase obtained or derived from *E. coli* poly(A) polymerase *(E. coli* PAP1) or *Saccharomyces cerevisiae* poly(A) polymerase (S. *cerevisiae* PAP1). In non-limiting embodiments, the cyclase catalyzes synthesis of RNA 2',3'-cyclic phosphate ends and catalyzes adenylylation of 5'-phosphate ends of the plurality of RNA fragments. The cyclase comprises RtcA. In embodiments, the method also comprises ligating oligonucleotide adapters to the 5' ends of the plurality of the 5' mono-adenylated RNA fragments, which may be performed using a T4 RNA ligase.

Thus, in a representative embodiment, the described approach provides a redesigned protocol for cDNA library construction (Fig. 1B). An approach to sequencing Ribosome Protected Fragments (RPFs) is shown, but can be adapted for use with any other type of RNA. In particular, instead of using 3' end linker ligation and circularization, the disclosure provides an enzymatic system capable of applying 3' end poly(A) tailing and 5'-end adenylation for the same RNA fragment. A specially designed 5' oligonucleotide permits highly efficient adapter ligation to the adenylated RPFs. By taking advantage of the biotinylated oligonucleotides for reverse transcription, the entire procedure can be accomplished within a single tube with minimal non-specific products. Compared to the standard Ribo-seq, Ezra-seq dramatically reduced the amount of starting material (~1 ng RNA), shortened the entire library processing time from 4 days to ~4 hr, and increased the resolution of RPFs with an averaged IFR >90%. From the same original sample, Ezra-seq nearly doubles the amount of RPFs with perfect reading frame (**Fig. 1A**, bottom panel). The superior resolution is highly reproducible and achievable from different cell types, including solid tissues. In embodiments, at least 80% of the 5' ends of the plurality of RNA fragments that are processed according to the method are sequenced. In certain and non-limiting embodiments, such as in the case of sequencing ribosome-protected RNA fragments, the disclosure provides for determining sequences that have an in-frame ratio (IFR) of at least 90% for sequenced RNA polynucleotides. As explained further below, the described approach to Ribo-seq is adaptable to determine the sequence of any type of RNA polynucleotides.

For use in performing the described methods, and for including in kits and articles of manufacture, the disclosure also provides a composition comprising a mixture of two distinct proteins, or a fusion protein, for use in RNA sequencing, the two distinct proteins or the fusion protein comprising a poly(A) polymerase and an RNA 3'-phosphate cyclase, wherein the cyclase comprises an RtcA enzyme. Compositions comprising such a fusion protein or a mixture of proteins are also provided. The disclosure includes isolated fusion protein comprising a poly(A) polymerase and an RNA 3'-phosphate cyclase.

In an aspect, the disclosure provides a kit comprising a mixture of the two described distinct proteins or a fusion protein, wherein the kit may also contain at least one of an RNA ligase or an RNA kinase. The kit may also comprise at least one container that contains at least the mixture of the two distinct proteins or the fusion protein. Any container may be used, such as vials, jars, sealable tubes, and the like. The kit may further include at least one oligonucleotide primer for use in cDNA synthesis. In general, the oligonucleotide primer contains a poly-T segment. The primer may be labeled so that it can bind to a binding partner. In one embodiment, the label comprises biotin. The kit may also comprise beads that include a moiety configured to bind to the label. In an embodiment, the moiety is streptavidin. Any suitable beads may be used, and are commercially available. In embodiments, beads comprise magnetic beads. The kit can also include a suitable buffer for use in RNA sequencing. In embodiments, the buffer has a pH of approximately 7.0, and/or an ATP concentration that is greater than 1 mM, and is optionally approximately 2 mM.

The disclosure also provides articles of manufacture, which include least one sealed container, which may contain the same or similar components as the described kits. The article of manufacture may also contained printed material and labeling that provides the components are used for RNA sequencing, and may include instructions for using the kit components.

### BRIEF DESCRIPTION OF FIGURES

**Fig. 1****.** Schematic representation **(A**) of Ezra-seq and conventional Ribo-seq methods. A direct comparison of the results in terms of IFR resolution is listed below. IFR: in-frame ratio of ribosome footprints. (**B**) The workflow of Ezra-seq for the application to ribosome profiling. RPF: ribosome-protected RNA fragments. (**C**) An overall procedure of single tube reaction using Ezra enzymes.
**Fig. 2****.** RtcA catalyzes the synthesis of RNA 2',3'-cyclic phosphate ends via an ATP-dependent pathway. After pre-treatment with T4 PNK, RtcA catalyzes ligase-like adenylylation of RNA 5'-monophosphate ends.
**Fig. 3****.** Different buffers were tested for the Ezra system (RtcA + PAP1), shown in (**A**)**.** P indicates positive control for separated steps. (**B**) New buffers were tested for the Ezra system (RtcA + PAP1) with different ATP concentration. P indicates positive control for separated steps. (**C**) The optimized buffer for the Ezra system.
**Fig. 4****.** The recombinant Ezra enzyme comprises PAP1 and RtcA, as shown in (**A**), enabling 5' end adenylation and 3' end polyadenylation for RNA molecules with 5' monophosphate and 3' OH. (**B**) Polyadenylation efficiency between yeast PAP1 and *E. coli* PAP1.
**Fig. 5****.** The full sequence of Biotin RT-primer (SEQ ID NO:5) is shown in (**A**). (**B**) The full sequence of 5' adapter for ligation (SEQ ID NO:2). (**C**) Ligation efficiency between 5' adapters with varied 3' ribonucleotides and AppRNA catalyzed by T4 RNL2.
**Fig. 6****.** Ribo-seq of MEF cells using Ezra-seq technology coupled with sucrose gradient-based ribosome fractionation is shown in (**A**)**.** (**B**) Ribo-seq of MEF cells using Ezra-seq technology without sucrose gradient-based ribosome fractionation. (**C**) Mitochondrial Ribo-seq and RNA-seq using Ezra-seq technology. (**D**) Chromatin-associated RNA-seq using Ezra-seq technology.
**Fig. 7****.** Graphical depiction of 3' & 5' adenylation.
**Fig. 8****.** Graphical depiction of bead binding.
**Fig. 9****.** Graphical depiction of ligation.
**Fig. 10****.** Graphical depiction of cDNA synthesis.
**Fig. 11****.** Graphical depiction of PCR amplification.

### DETAILED DESCRIPTION

Unless specified to the contrary, it is intended that every maximum numerical limitation given throughout this description includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The disclosure includes every amino acid sequence described herein, and every polynucleotide sequence that encodes the amino acid sequences, including but not limited to cDNA sequences, and RNA sequences. Complementary sequences, and reverse complementary sequences are also included. Expression vectors comprising such nucleotide sequences are encompassed by the disclosure.

Polypeptides comprising amino acid sequences that are at least 80% identical to the amino acid sequence of this disclosure are included. In embodiments, the proteins comprise mutations, relative to an endogenous protein. An "endogenous" protein is a protein that is normally encoded by an unmodified gene. Likewise, an endogenous gene or other polynucleotide comprises a DNA sequence that is unmodified, such as by recombinant, gene editing, or other approaches. Mutations can include amino acid insertions, deletions, and changes.

In embodiments, the disclosure provides compositions and methods for RNA adenylation and sequencing. The method is referred to from time to time as Ezra-seq, which stands for easy **R**NA-**a**denylation **seq**uencing. The term "easy" should be viewed in the context of the disclosure, which provides novel compositions and methods for sequencing RNA with previously unavailable efficiency and resolution, but is not intended to signify a simplistic nature of the disclosure.

In general, the disclosure provides compositions and methods for RNA-associated sequencing, wherein the RNA fragment is modified with a 3' end poly(A) tailing and 5'-end adenylation, followed by direct amplification. The described modifications are achieved enzymatically (e.g., enzymes) as opposed to chemical modification performed without enzymes.

In embodiments, methods of the disclosure can be provided with or without using fusion proteins, such as by using a mixture of different enzymes. Thus, in one aspect, the disclosure provides one or more fusion proteins that are suitable for use in the described RNA modification methods, which include but are not necessarily limited to 5' and 3' adenylation of RNA. A fusion protein comprises a single, contiguous polypeptide, with segments of distinct proteins within the fusion protein. In embodiments, a fusion protein of the disclosure is referred to as an "Ezra" enzyme, which stands for **e**a**s**y **R**NA-**a**denylation enzyme. Thus, in embodiments, all the enzymes used in the described compositions, methods and kits may be separate proteins, or some of the enzymes may be present in at least one fusion protein. In embodiments, at least two of the described enzymes may be present in a fusion protein. In embodiments, a fusion protein comprises a segment that is a cyclase and a segment that is a polymerase. In embodiments, the cyclase comprises an RNA 3'-phosphate cyclase that catalyzes the synthesis of RNA 2',3'-cyclic phosphate ends and also catalyzes adenylylation of 5'-phosphate ends of RNA strands. In embodiments, the described proteins are obtained or derived from prokaryotes, e.g., bacteria, or eukaryotes, e.g., yeasts. In embodiments, the polymerase, which may be used as a distinct protein or as a component of a fusion protein, comprises a poly(A) polymerase. The poly(A) polymerase may be isolated or derived from a prokaryotic or eukaryotic source. "Derived from" means the endogenously produced protein may be modified, such as to include a purification tag, or one or more change in the amino acid sequence, provided the protein retains its enzymatic function. In embodiments, the described proteins include any suitable purification tag, including but not necessarily limited to a polyhistidine tag, typically containing 2-10 histidines, a Strep-tag, Small Ubiquitin-like Modifier (SUMO), Maltose Binding Protein (MBP) tag, N-terminal glutathione S-transferase (GST), and the like.

In an embodiment, the poly(A) polymerase is an *E. coli* poly(A) polymerase or a *Saccharomyces cerevisiae* poly(A) polymerase. Representative and non-limiting embodiments of such enzymes are provided as an *E. coli* poly(A) polymerase (PAP1) and *Saccharomyces cerevisiae* poly(A) polymerase (PAP1). The cyclase is *E. coli* RtcA. In embodiments, functional segments of enzymes described herein can be used. Functional segments comprise a segment of the described enzyme that is necessary and sufficient to perform its intended function, the functions of the described enzymes being further described herein and illustrated in certain figures.

In connection with the present disclosure, it is known that high through-put sequencing (HTS) of RNA fragments generally requires the preparation of libraries where the RNA is placed between the known 5'- and 3'-terminal sequences. Prior to the present disclosure, available methods for library construction utilized either RNA or DNA adaptor ligation to the 5'- and 3'- ends of the target RNA molecules. The adaptors provide primer annealing sites, first for the reverse transcription (RT) primer and later for the polymerase chain reaction (PCR) and HTS sequencing. However, ligation of adaptors in this manner is not only time consuming but also a low efficiency process that requires micrograms of inputs. In addition, the resulting cDNA libraries are contaminated with cross- and self-ligation adaptor by-products and require additional purification steps both before and after pre-amplification. Additionally, small RNAs with 5' recessed ends are poor substrates for enzymatic adapter ligation (8).

Many previously available small RNA sequencing protocols use synthesized DNA oligonucleotide adapters with 5' preadenylation during cDNA library preparation. Preadenylation of the adapter's 5' end facilitates the ligation of the adapter to the 3' end of RNA molecules without the addition of ATP, thereby avoiding ATP-dependent side reactions. However, preadenylation of the DNA adapters can be costly and difficult. The previously available methods for chemical adenylation of DNA adapters is inefficient and requires additional steps for purification. An alternative enzymatic method using a commercial RNA ligase was recently introduced, but this enzyme works best as a stoichiometric adenylating reagent rather than a catalyst (*9*). Thus, in embodiments, the disclosure includes the proviso that adenylation of RNA is not performed using a pre-adenylated oligonucleotide. Rather, adenylation is enzymatically performed directly on RNA polynucleotides, including but not limited to fragments of RNA polynucleotides.

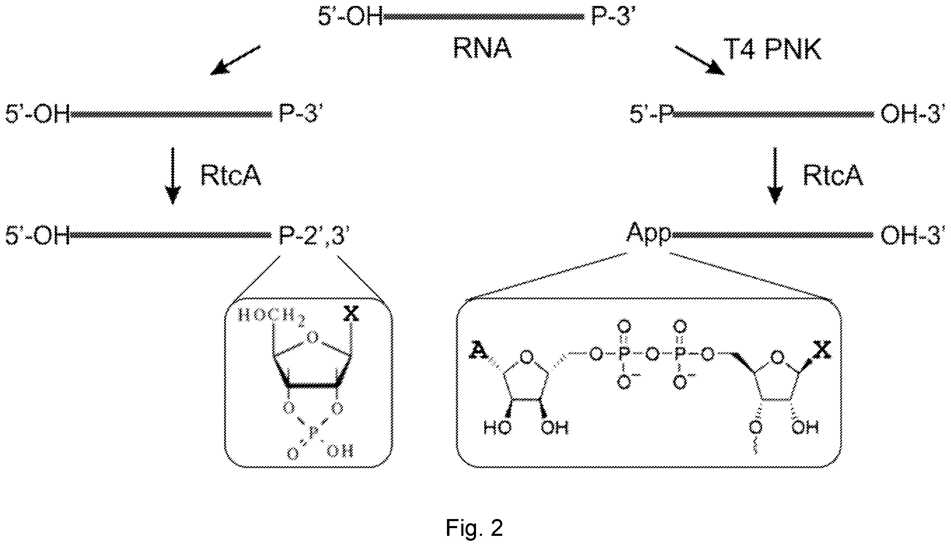

The present disclosure demonstrates use of an RNA 3'-phosphate cyclase (RtcA) that not only catalyze the synthesis of RNA 2',3'-cyclic phosphate ends, but also catalyzes adenylylation of 5'-phosphate ends of RNA strands (**Fig. 2**). The adenylylation results in the "App" structure shown in Fig. 2, showing a single A with two phosphates. This adenylylation may also be referred to as adenylation, as is often the case in the art. The disclosure includes but is not limited to all enzymatic modifications of RNA shown in Fig. 2.

When RNA fragments are pretreated with a suitable kinase that phosphorylates 5' ends but dephosphorylate 3' ends, the RNA fragments become active "linkers" once the 5' end is adenylylated by RtcA. A representative and non-limiting example of a suitable kinase is illustrated herein as T4 polynucleotide kinase.

For RNA fragments to be sequenced, both 5' and 3' adaptors are required for library preparation. RNA fragments as used herein may be any suitable size, non-limiting embodiments of which include RNA polynucleotides having a minimal length of approximately 20 nucleotides. In embodiments, the length is 20-100 nts, but shorter or longer polynucleotides are not excluded from the scope of the disclosure. Thus, in embodiments, an RNA fragment can include an RNA polynucleotide that has not necessarily been fragmented, such as by mechanical fragmentation. While the disclosure is suitable for sequencing intact RNA, such as intact mRNA, or small RNAs, such as certain miRNAs and tRNA, in embodiments, the disclosure pertains to sequencing RNA polynucleotides that have been fragmented. Generating RNA fragments can be achieved using any suitable technique, which generally involve mechanical disruption of intact RNA polynucleotides. Suitable methods include but are not limited to sonication, acoustic shearing, hydrodynamic shearing, but alternative methods can be used, such as heat and divalent metal cation exposure.

Standard protocols use separate steps and require additional purification for ligated products. Compared to the commonly used 3' linker ligation, polyadenylation at the 3' end is efficient and free of bias. In the presence of ATP, the poly(A) polymerase (PAP1) from*E*. *coli* or *Saccharomyces cerevisiae* adds a poly(A) tail to the RNA fragments. Since both 5' adenylylation and 3' polyadenylation require ATP molecules and occur at the same temperature (37°C), the present disclosure provides for combining these two reactions into a single reaction (e.g., Ezra). The system can greatly simplify the entire procedure by shortening the processing time from 2 hours to 30 min. Importantly, the described method prevents product loss by omitting purification steps, such as ethanol precipitation. Thus, in an embodiment, a method of the disclosure may be free of ethanol precipitation, or precipitation by other solvents.

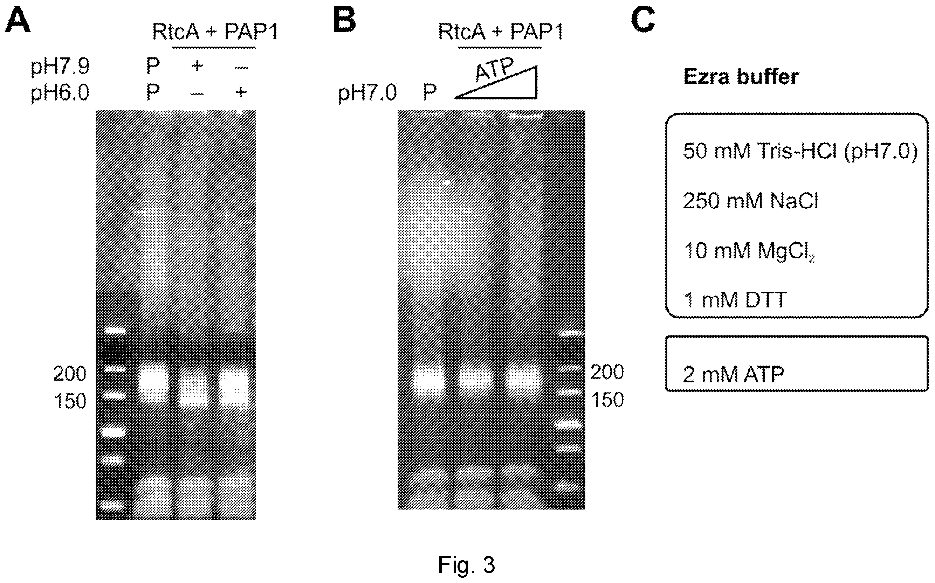

However, it is revealed in the present disclosure that the working buffers for PAP1 and RtcA enzymes are not compatible. Specifically, the optimal buffer for PAP1 has a pH 7.9, whereas RtcA works the best at pH 6.0. After a titration of pH values, we found that pH 7.0 works for both PAP1 and RtcA (Fig. 3A). Additionally, by increasing the ATP concentration from 1 mM to 2 mM, we obtained a higher efficiency (Fig. 3B). With these novel buffer conditions (Fig. 3C), the disclosure provides an Ezra system capable of 5' adenylylation and 3' polyadenylation for the same RNA samples. All of the described buffers are included within the scope of this disclosure.

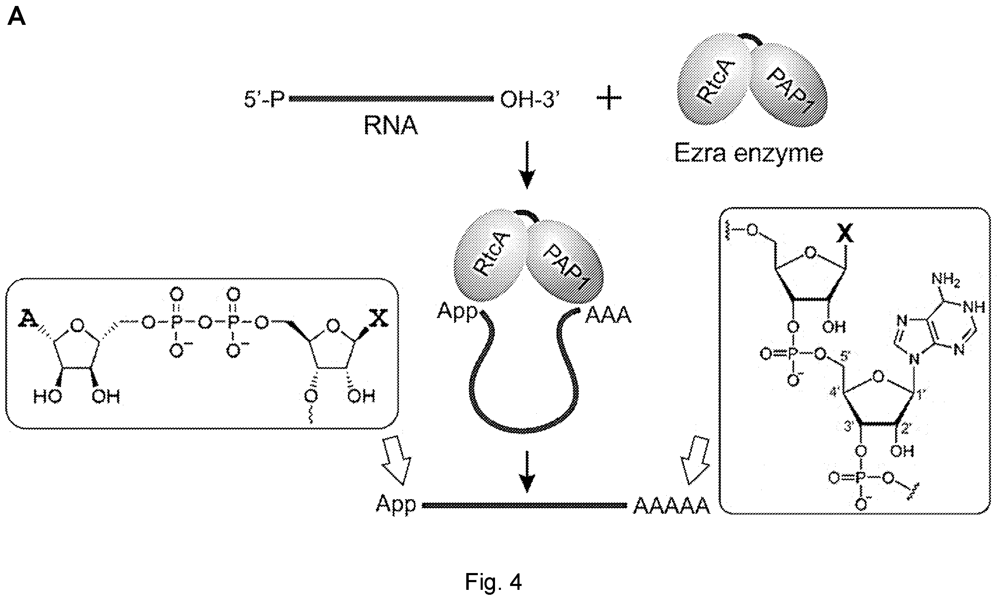

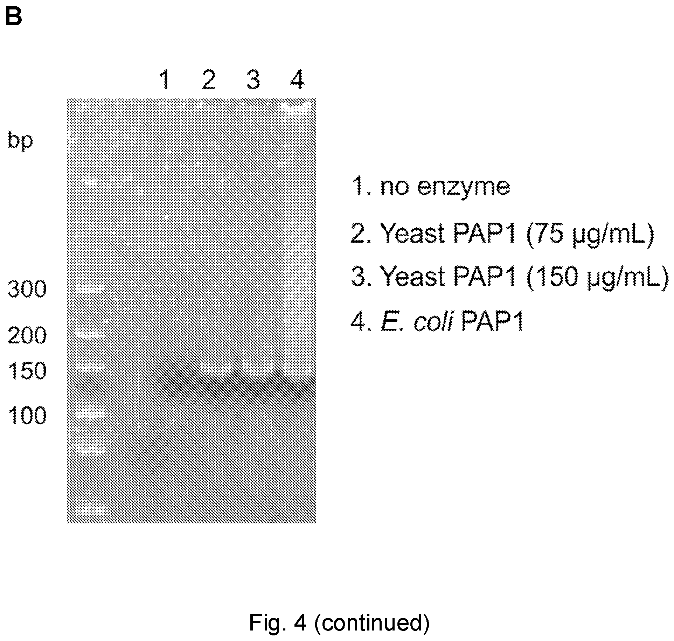

To increase the efficiency of RNA adenylation at both ends of the RNA fragments, we engineered several fusion proteins composed of RtcA and PAP1. The most active enzyme (PAP1-RtcA) with a XTEN linker was renamed as Ezra enzyme for easy RNA adenylases (Fig. 4). Therefore, the RNA 5'-adenylation and 3'-polyadenylation can be achieved in the same tube without purification. Thus, in embodiments, the cyclase and polymerase may be separated from one another within a fusion protein by any suitable linker, a non-limiting embodiment of which is the described XTEN linker. However, as is known in the art, suitable linkers can comprise varying lengths and varying amino acid sequences, and any suitable linker can be used to create a fusion protein of the cyclase and polymerase. In embodiments, linker can comprise from 1-20 amino acids, inclusive, and including all integers and ranges of integers there between. In embodiments, a flexible linker is used. In embodiments, linkers may include glycine and serine.

Further, while non-limiting demonstrations of the disclosure are shown with the polymerase N terminally located followed by the C terminally located cyclase, other configurations can be used, such as having the polymerase located C terminally relative to the cyclase.

In embodiments, the described compositions, methods, and kits may include two distinct proteins, or a fusion protein comprising the amino acid sequences of two distinct proteins. In an embodiment, the distinct proteins are RNA 3'-phosphate cyclase (RtcA) and a poly(A) polymerase. In a non-limiting embodiment, the poly(A) polymerase is *E. coli* poly(A) polymerase (PAP1) or *Saccharomyces cerevisiae* poly(A) polymerase (PAP1). Representative and non-limiting sequences of suitable cyclase and polymerase enzymes are described below.

An illustration of a representative Ezra fusion protein comprising PAP1 and RtcA is provided in **Fig. 4A****.** The activities of *E. coli* poly(A) polymerase (PAP1) and *Saccharomyces cerevisiae* poly(A) polymerase (PAP1) are similar (**Fig. 4B**). The Ezra fusion protein sequence is listed below, where the His-tag is shown in italics, the PAP1 sequence is shown in bold, the linker is subscripted, and the RtcA sequence is underlined. SEQ ID NO:1 is for Ezra fusion protein containing *E. coli* poly(A) polymerase (PAP1), whereas SEQ ID NO:2 is for *Saccharomyces cerevisiae* poly(A) polymerase (PAP1).

MHHHHHHHHHMKVLSREESEAEQAVARPQVTVIPREQHAISRKDISENALKVMY
RLNKAGYEAWLVGGGVRDLLLGKKPKDFDVTTNATPEQVRKLFRNCRLVGRR
FRLAHVMFGPEIIEVATFRGHHEGNVSDRTTSQRGQNGMLLRDNIFGSIEEDAQ
RRDFTINSLYYSVADFTVRDYVGGMKDLKDGVIRLIGNPETRYREDPVRMLRAV
RFAAKLGMRISPETAEPIPRLATLLNDIPPARLFEESLKLLQAGYGYETYKLLCE
YHLFQPLFPTITRYFTENGDSPMERIIEQVLKNTDTRIHNDMRVNPAFLFAAMF
WYPLLETAQKIAQESGLTYHDAFALAMNDVLDEACRSLAIPKRLTTLTRDIWQL
QLRMSRRQGKRAWKLLEHPKFRAAYDLLALRAEVERNAELQRLVKWWGEFQ
VSAPPDQKGMLNELDEEPSPRRRTRRPRKRAPRREGTASGSETPGTSESATPESHMMKR
MIALDGAQGEGGGQILRSALSLSMITGLPFTITGIRAGRAKPGLLRQHLT
AVKAAAEICRATVEGAELGSQRLLFRPGTVRGGDYRFAIGSAGSCTLVL
QTVLPALWFADGPSRVEVSGGTDNPSAPPADFIRRVLEPLLAKIGIHQQT
TLLRHGFYPAGGGVVATEVSPVTSFNTLQLGERGNIVRLRGEVLLAGVP
RHVAEREIATLAASFSLHEQNIHNLPRDQGPGNTVSLEVESENITERFFV
VGEKRVSAEVVAAQLVKEVKRYLASPAAVGEYLADQLVLPMALAGAG
EFTVAHPSCHLLTNIAVVERFLPVRFGLVEADGVTRVSIE* (SEQ ID NO:1)

Referring to SEQ ID NO:1, amino acids 1-11 correspond to a poly-His affinity tag, amino acids 12-466 correspond to *E. coli* PAP1, amino acids 467-484 correspond to a XTEN linker, and amino acids 485-822 correspond to RtcA.

In embodiments, a method of the disclosure is performed using a contiguous polypeptide that comprises amino acid sequences that are at least 80% identical to segment of SEQ ID NO:1 that includes amino acids 12-466 and amino acid sequences that are at least 80% similar to segment of SEQ ID NO:1 that includes amino acids 485 to 822.

MHHHHHHHHMSSQKVFGITGPVSTVGATAAENKLNDSLIQELKKEGSFETEQE
TANRVQVLKILQELAQRFVYEVSKKKNMSDGMARDAGGKIFTYGSYRLGVHGP
GSDIDTLVVVPKHVTREDFFTVFDSLLRERKELDEIAPVPDAFVPIIKIKFSGISIDL
ICARLDQPQVPLSLTLSDKNLLRNLDEKDLRALNGTRVTDEILELVPKPNVFRIA
LRAIKLWAQRRAVYANIFGFPGGVAWAMLVARICQLYPNACSAVILNRFFIILSE
WNWPQPVILKPIEDGPLQVRVWNPKIYAQDRSHRMPVITPAYPSMCATHNITES
TKKVILQEFVRGVQITNDIFSNKKSWANLFEKNDFFFRYKFYLEITAYTRGSDEQ
HLKWSGLVESKVRLLVMKLEVLAGIKIAHPFTKPFESSYCCPTEDDYEMIQDKY
GSHKTETALNALKLVTDENKEEESIKDAPKAYLSTMYIGLDFNIENKKEKVDIHI
PCTEFVNLCRSFNEDYGDHKVFNLALRFVKGYDLPDEVFDENEKRPSKKSKRKN
LDARHETVKRSKSDAASGDNINGTTAAVDVNSGSETPGTSESATPESHMMKRMIALDGA
QGEGGGQILRSALSLSMITGLPFTITGIRAGRAKPGLLRQHLTAVKAAAE
ICRATVEGAELGSQRLLFRPGTVRGGDYRFAIGSAGSCTLVLQTVLPAL
WFADGPSRVEVSGGTDNPSAPPADFIRRVLEPLLAKIGIHQQTTLLRHGF
YPAGGGVVATEVSPVTSFNTLQLGERGNIVRLRGEVLLAGVPRHVAER
EIATLAASFSLHEQNIHNLPRDQGPGNTVSLEVESENITERFFVVGEKRV
SAEVVAAQLVKEVKRYLASPAAVGEYLADQLVLPMALAGAGEFTVAH
PSCHLLTNIAVVERFLPVRFGLVEADGVTRVSIE* (SEQ ID NO:2)

Referring to SEQ ID NO:2, amino acids 1-11 correspond to a an affinity tag, amino acids 12-578 correspond to *Saccharomyces cerevisiae* PAP1, amino acids 579- 596 correspond to a XTEN linker, and amino acids 597-934 correspond to RtcA.

In embodiments, a method of the disclosure is performed using a contiguous polypeptide that comprises amino acid sequences that are at least 80% identical to segment of SEQ ID NO:2 that includes amino acids 12-578 and amino acid sequences that are at least 80% similar to segment of SEQ ID NO:2 that includes amino acids 597 to 934.

A representative and non-limiting DNA sequence encoding the Ezra fusion protein is shown below, as SEQ ID NO:3 for *E*. *coli* poly(A) polymerase (PAP1), and SEQ ID NO:4 is for *Saccharomyces cerevisiae* poly(A) polymerase (PAP1), using the same convention for the coding sequences as in the amino acid sequence above:

ATGCATCATCATCATCATCATCATCATCATATGAAAGTGCTGAGCCGCGAAGAAAGCGAAGCGG
AACAGGCGGTGGCGCGCCCGCAGGTGACCGTGATTCCGCGCGAACAGCATGCGATTAGCCGCAAAGAT
ATTAGCGAAAACGCGCTGAAAGTGATGTATCGCCTGAACAAAGCGGGCTATGAAGCGTGGCTGGTGGG
CGGCGGCGTGCGCGATCTGCTGCTGGGCAAAAAACCGAAAGATTTTGATGTGACCACCAACGCGACCC
CGGAACAGGTGCGCAAACTGTTTCGCAACTGCCGCCTGGTGGGCCGCCGCTTTCGCCTGGCGCATGTG
ATGTTTGGCCCGGAAATTATTGAAGTGGCGACCTTTCGCGGCCATCATGAAGGCAACGTGAGCGATCGC
ACCACCAGCCAGCGCGGCCAGAACGGCATGCTGCTGCGCGATAACATTTTTGGCAGCATTGAAGAAGA
TGCGCAGCGCCGCGATTTTACCATTAACAGCCTGTATTATAGCGTGGCGGATTTTACCGTGCGCGATTA
TGTGGGCGGCATGAAAGATCTGAAAGATGGCGTGATTCGCCTGATTGGCAACCCGGAAACCCGCTATC
GCGAAGATCCGGTGCGCATGCTGCGCGCGGTGCGCTTTGCGGCGAAACTGGGCATGCGCATTAGCCCG
GAAACCGCGGAACCGATTCCGCGCCTGGCGACCCTGCTGAACGATATTCCGCCGGCGCGCCTGTTTGA
AGAAAGCCTGAAACTGCTGCAGGCGGGCTATGGCTATGAAACCTATAAACTGCTGTGCGAATATCATCT
GTTTCAGCCGCTGTTTCCGACCATTACCCGCTATTTTACCGAAAACGGCGATAGCCCGATGGAACGCAT
TATTGAACAGGTGCTGAAAAACACCGATACCCGCATTCATAACGATATGCGCGTGAACCCGGCGTTTCT
GTTTGCGGCGATGTTTTGGTATCCGCTGCTGGAAACCGCGCAGAAAATTGCGCAGGAAAGCGGCCTGA
CCTATCATGATGCGTTTGCGCTGGCGATGAACGATGTGCTGGATGAAGCGTGCCGCAGCCTGGCGATT
CCGAAACGCCTGACCACCCTGACCCGCGATATTTGGCAGCTGCAGCTGCGCATGAGCCGCCGCCAGGG
CAAACGCGCGTGGAAACTGCTGGAACATCCGAAATTTCGCGCGGCGTATGATCTGCTGGCGCTGCGCG
CGGAAGTGGAACGCAACGCGGAACTGCAGCGCCTGGTGAAATGGTGGGGCGAATTTCAGGTGAGCGCG
CCGCCGGATCAGAAAGGCATGCTGAACGAACTGGATGAAGAACCGAGCCCGCGCCGCCGCACCCGCCG
CCCGCGCAAACGCGCGCCGCGCCGCGAAGGCACCGCGAGCGGCAGCGAGACTCCCGGGACCTCAGAGTCCGCC
ACACCCGAAAGTCATATGATGAAAAGGATGATTGCGCTGGATGGCGCACAGGGC
GAAGGCGGCGGGCAGATCCTGCGCTCGGCGCTGAGCCTGTCGATGA
TAACCGGCCTGCCATTTACCATCACCGGCATTCGTGCCGGGCGGGCA
AAACCGGGACTGTTGCGCCAGCATCTGACCGCGGTAAAAGCGGCTG
CGGAAATTTGTAGGGCAACGGTGGAAGGTGCGGAGCTGGGATCGCA
GCGTCTGCTCTTCCGGCCCGGCACCGTGCGCGGCGGCGATTACCGCT
TTGCTATCGGTAGCGCCGGAAGTTGTACGCTGGTGCTGCAAACGGTG
CTGCCCGCGCTGTGGTTTGCCGATGGACCTTCGCGTGTTGAAGTGAG
CGGAGGCACCGATAACCCGTCGGCCCCGCCTGCGGATTTTATCCGCC
GGGTGCTGGAGCCGCTGCTGGCGAAAATAGGAATTCATCAGCAAAC
CACGCTGTTACGTCACGGTTTTTATCCTGCCGGAGGCGGCGTGGTGG
CAACGGAAGTCTCGCCGGTGACATCGTTTAACACCTTGCAACTTGGC
GAGCGCGGGAACATTGTGCGGCTGCGTGGTGAGGTGTTATTAGCTGG
CGTACCGCGACATGTTGCTGAGCGTGAAATCGCTACGCTGGCGGCAA
GTTTTTCCCTGCATGAGCAGAATATTCATAACCTGCCGCGTGACCAG
GGGCCGGGTAATACCGTTTCGCTTGAAGTCGAAAGTGAAAATATCAC
CGAACGCTTTTTTGTCGTCGGTGAAAAGCGCGTCAGCGCCGAGGTGG
TCGCGGCACAGTTGGTGAAAGAGGTGAAACGCTACCTGGCAAGCCC
GGCGGCGGTGGGGGAATATCTCGCCGACCAGTTGGTGCTACCGATG
GCGCTGGCGGGCGCGGGAGAATTTACGGTCGCCCATCCCTCATGCCA
TCTGCTGACCAATATCGCGGTGGTGGAGCGTTTCTTGCCAGTGCGGT
TTGGTCTGGTGGAGGCTGATGGCGTAACGCGGGTGAGCATTGAATA
A (SEQ ID NO:3)

ATGCATCATCATCATCATCATCATCATCATATGAGCTCTCAAAAGGTTTTTGGTATTACTGGACC
TGTTTCCACCGTGGGCGCCACAGCAGCAGAAAATAAATTAAATGATAGTTTAATCCAAGAACTGAAAAA
GGAAGGATCGTTCGAAACAGAGCAAGAAACTGCCAATAGGGTACAAGTGTTGAAAATATTGCAGGAATT
GGCACAAAGATTTGTTTATGAAGTATCGAAGAAGAAAAATATGTCAGACGGGATGGCAAGGGATGCTG
GTGGGAAGATTTTTACGTATGGGTCCTATAGACTAGGAGTCCATGGGCCTGGTAGTGATATCGATACTT
TGGTAGTTGTTCCAAAACATGTAACTCGGGAAGATTTTTTTACGGTATTTGATTCACTACTGAGAGAGA
GGAAGGAACTGGATGAAATTGCACCTGTACCTGATGCGTTTGTCCCGATTATCAAGATAAAGTTCAGTG

GTATTTCTATCGATTTAATCTGTGCACGTCTAGACCAACCTCAAGTGCCTTTATCCTTGACTTTATCAGA
TAAAAATCTACTGCGAAATCTAGACGAGAAGGACTTGAGAGCTTTGAATGGTACCAGAGTAACAGATGA
GATATTAGAACTGGTACCAAAGCCGAATGTTTTCAGAATCGCTTTAAGAGCTATTAAGCTATGGGCCCA
AAGAAGGGCTGTTTATGCTAATATTTTTGGTTTTCCTGGTGGTGTGGCTTGGGCCATGCTAGTGGCTAG
AATTTGTCAACTATACCCTAACGCCTGTAGCGCAGTTATATTGAACAGATTTTTCATCATTTTGTCGGAA
TGGAATTGGCCACAACCTGTTATCTTGAAACCAATTGAGGATGGCCCGTTACAAGTTCGTGTATGGAAT
CCAAAGATATATGCCCAAGACAGGTCTCATAGAATGCCCGTCATTACACCAGCTTATCCATCAATGTGT
GCTACCCATAACATCACGGAATCTACTAAAAAAGTCATTTTACAGGAATTCGTAAGAGGCGTTCAAATT
ACGAATGATATTTTTTCCAATAAGAAGTCCTGGGCCAATTTATTCGAAAAAAACGATTTTTTCTTTCGAT
ACAAGTTCTATTTAGAAATTACTGCATATACAAGGGGCAGTGACGAGCAGCATTTAAAATGGAGTGGTC
TTGTTGAAAGTAAGGTAAGGCTTCTAGTTATGAAACTGGAGGTGTTAGCTGGAATAAAAATTGCACATC
CTTTCACCAAACCCTTTGAAAGTAGTTATTGTTGTCCAACCGAGGATGACTATGAAATGATTCAAGACAA
ATACGGTAGTCATAAAACTGAGACAGCACTGAACGCCCTTAAACTGGTAACAGATGAAAATAAAGAGGA
AGAAAGTATTAAAGATGCACCAAAGGCATATTTAAGCACCATGTACATAGGCCTTGACTTTAATATTGA
AAACAAAAAGGAAAAAGTTGACATTCACATTCCCTGCACTGAATTTGTGAATTTATGTCGAAGTTTCAAT
GAGGATTATGGTGACCACAAAGTATTCAATCTAGCCCTCCGCTTCGTAAAGGGTTACGATTTGCCAGAT
GAAGTTTTCGATGAAAATGAAAAGAGACCATCAAAGAAGAGTAAAAGGAAGAATTTAGATGCTAGACAT
GAAACCGTGAAGAGATCTAAATCAGATGCTGCTTCAGGTGACAACATCAATGGCACAACCGCAGCTGTT
GACGTAAACAGCGGCAGCGAGACTCCCGGGACCTCAGAGTCCGCCACACCCGAAAGTCATATGATGAAAAGG
ATGATTGCGCTGGATGGCGCACAGGGCGAAGGCGGCGGGCAGATCC
TGCGCTCGGCGCTGAGCCTGTCGATGATAACCGGCCTGCCATTTACC
ATCACCGGCATTCGTGCCGGGCGGGCAAAACCGGGACTGTTGCGCC
AGCATCTGACCGCGGTAAAAGCGGCTGCGGAAATTTGTAGGGCAAC
GGTGGAAGGTGCGGAGCTGGGATCGCAGCGTCTGCTCTTCCGGCCCG
GCACCGTGCGCGGCGGCGATTACCGCTTTGCTATCGGTAGCGCCGGA
AGTTGTACGCTGGTGCTGCAAACGGTGCTGCCCGCGCTGTGGTTTGC
CGATGGACCTTCGCGTGTTGAAGTGAGCGGAGGCACCGATAACCCG
TCGGCCCCGCCTGCGGATTTTATCCGCCGGGTGCTGGAGCCGCTGCT
GGCGAAAATAGGAATTCATCAGCAAACCACGCTGTTACGTCACGGTT
TTTATCCTGCCGGAGGCGGCGTGGTGGCAACGGAAGTCTCGCCGGTG
ACATCGTTTAACACCTTGCAACTTGGCGAGCGCGGGAACATTGTGCG
GCTGCGTGGTGAGGTGTTATTAGCTGGCGTACCGCGACATGTTGCTG
AGCGTGAAATCGCTACGCTGGCGGCAAGTTTTTCCCTGCATGAGCAG
AATATTCATAACCTGCCGCGTGACCAGGGGCCGGGTAATACCGTTTC
GCTTGAAGTCGAAAGTGAAAATATCACCGAACGCTTTTTTGTCGTCG
GTGAAAAGCGCGTCAGCGCCGAGGTGGTCGCGGCACAGTTGGTGAA
AGAGGTGAAACGCTACCTGGCAAGCCCGGCGGCGGTGGGGGAATAT
CTCGCCGACCAGTTGGTGCTACCGATGGCGCTGGCGGGCGCGGGAG
AATTTACGGTCGCCCATCCCTCATGCCATCTGCTGACCAATATCGCG
GTGGTGGAGCGTTTCTTGCCAGTGCGGTTTGGTCTGGTGGAGGCTGA
TGGCGTAACGCGGGTGAGCATTGAATAA (SEQ ID NO:4)

As discussed above, aspects of the disclosure are illustrated using ribosome protected fragments (RFPs), but the same approach can be adapted to other RNA fragments, with the exception that if RFPs are used there is generally no requirement to remove ribosomal RNA. In this regard, a comparison of the Ezra-seq for sequencing RFPs, as a non-limiting example of a type of RNA that can be sequenced according to the present disclosure, and conventional "Ribo-seq" methods is provided schematically in Fig. 1A. A non-limiting depiction of a method of this disclosure is provided schematically in Fig. 1B. As depicted in Fig. 1B, the disclosure provides for sequencing a plurality of RNA polynucleotides. The method generally comprises: 1) contacting a plurality of RNA polynucleotides with one or more enzymes and oligonucleotides as described further below, such that the RNA polynucleotides are subjected to 5'-adenylation and 3'-polyadenylation, and 2) amplifying the RNA polynucleotides into cDNAs, which facilitates the sequence of the RNA polynucleotides.

The type of RNA sequenced using the compositions and methods described herein is not particularly limited. In embodiments, the RNA is produced by a prokaryote, a eukaryote, or a virus. In embodiments, the RNA polynucleotides sequenced according to this disclosure include but are not limited to messenger RNA (mRNA), as described above. In embodiments, the mRNA may be fragmented so that segments of the mRNA that do not already have a poly-A tail are sequenced. Any RNA that is sequenced may also be fragmented, if desired. RNA that can be sequenced also includes transfer RNA (tRNA), ribosomal RNA (rRNA), Transfer-messenger RNA (tmRNA), small nuclear RNA (snRNA), any type of antisense RNA, ribozymes, microRNA (miRNA), small interfering RNA (siRNA), short hairpin RNA (shRNA), RNA viral genomes, any CRISPR RNA, including but not limited to guide RNA and trans-activating crRNA, double stranded RNA (dsRNA), and any other type of RNA, irrespective of whether or not the RNA contains an open reading frame, or has a known or unknown function. The RNA may be located in the nucleus or the cytoplasm of a cell, or it may be excreted from a cell, such being within RNA-containing secreted exosomes. In embodiments, RNA polynucleotides sequenced according to the disclosure comprises one or more N6 methyl adenosines. In embodiments, nascent, actively transcribed RNAs are sequenced. In embodiments, the compositions and methods described herein are adapted to be used with any existing or later developed RNA sequencing approaches. Non-limiting examples of existing approaches include RIP-seq (RNA immunoprecipitation), CLIP-seq (Cross-linking immunoprecipitation), ChIP-seq (chromatin-immunoprecipitation), as well as genome-wide detection of RNA modifications (for instance, m⁶A-seq, as described above).

In embodiments which pertain to sequencing RPFs, segments of RNA that are protected by ribosomes from nuclease digestion are sequenced. Thus, in embodiments, ribosome-protected fragments of mRNA are sequenced. In one embodiment, the entire set of ribosome-protected mRNA RPFs from a sample are sequenced. In embodiments, the compositions and methods are thus suitable for use in, for example, Ribosome profiling (referred to herein from time to time as "Ribo-seq"). In embodiments, the disclosure provides for an RNA sequencing approach such that ribosome positions and/or density across the transcriptome at a sub-codon resolution is provided. In embodiments, the disclosure results in a higher in-frame ratio of ribosome footprints, relative to out of frame footprints, wherein a ribosome "footprint" means the segment of an RNA polynucleotide that is protected from enzymatic degradation by a ribosome. By "in-frame" it is meant that the order of codons in the RNA is intact in the 0 frame starting with the first nucleotide in the sequenced RNA. In embodiments, Ribo-seq as described herein is performed without sucrose gradient-based ribosome separation. In embodiments, Ribo-seq can be performed using whole cell lysates to provide the RNA fragments.

In embodiments, as an alternative to using 3' end linker ligation and circularization, the presently provided disclosure provides for 3' end poly(A) tailing and 5'-end adenylation on the same RNA fragment. In embodiments, 5'-adenylated RNA is referred to as AppRNA. In embodiments, by using the compositions and methods of this disclosure, RNA 5'-adenylation and 3'-polyadenylation can be achieved in a single reaction vessel without a purification step, such as purification of RNA polynucleotides or DNA polynucleotides, including but not limited to oligonucleotides, primers, and the like.

The disclosure includes all reagents described herein, and combinations of reagents. The disclosure includes all concentrations of components as described herein, representative and non-limiting examples of which include buffers, pH values, nucleotide, RNA, and enzyme concentrations, volumes, and any other quantitative value described herein. The disclosure includes all time periods, temperatures, and value intervals. In non-limiting examples, a method of the disclosure is performed in a solution having a pH of approximately from 6.0 to 7.9, inclusive, and including all numbers there between to the first decimal point. In embodiments, a method of the disclosure is performed in a solution having a pH of approximately or precisely 7.0. In embodiments, the ATP concentration in a solution of the disclosure is greater than 1 mM. In embodiments, the ATP concentration in a solution of the disclosure is approximately or precisely 2 mM. In embodiments, the disclosure provides a buffer comprising approximately 50 mM Tris-HCL, 250 mM NaCl, 10 mM MgCl₂, 1 mM DTT and 2 mM ATP. In embodiments, the sequence of a plurality of RNA polynucleotides is performed in a period of time that does not exceed approximately 8 hours. In embodiments, a cDNA library is produced in a period of time that does not exceed approximately 2 hours. In embodiments, a cDNA library is produced in a period of approximately 30 minutes.

In embodiments, an RNA sequencing process described herein is performed using a sample comprising as little as approximately 1 nanogram of RNA. Thus, in embodiments, picogram amounts of RNA from a sample are sequenced. In embodiments, picogram amounts of RNA fragments are sequenced with ultra-resolution. In an embodiment, ultra-resolution comprises resolution of RPFs with an average IFR >90% as a fraction of the total fragments sequenced.

In embodiments, the disclosure provides for RNA sequencing without template switching, e.g., template-switching polymerase chain reaction (TS-PCR). Thus, the disclosure is different and improved relative to the procedure offered by CLONTECH as Switching Mechanism At the 5' end of RNA Template (SMART), and by DIAGENODE as Capture and Amplification by Tailing and Switching (CATS).

In embodiments, RNA sequencing results produced by using the described compositions and methods are not biased by the presence of a G nucleotide in the 5' of the RNA polynucleotides. Thus, in embodiments, the disclosure provides for sequencing a plurality of RNA polynucleotides that have 5' nucleotides that are distributed randomly and/or without a discernable 5' end nucleotide pattern across said plurality.

In embodiments, a method of this disclosure provides for increased accuracy of RNA 5' end sequencing. In embodiments, 5' ends of 80-90% of RNA polynucleotides in a sample are sequenced. In embodiments, 5' ends of more than 90% of the RNA polynucleotides in a sample are sequenced. In embodiments, the disclosure provides 5' adapter ligation of polyadenylated RNA. In embodiments, the disclosure provides for producing a plurality of cDNAs, such as cDNA libraries, from RNA segments, wherein the plurality of cDNAs do not include cross- and self-ligation adaptor by-products, such as self-ligated adaptors and adaptor-RT primer ligation.

In embodiments, the disclosure includes the sequential or concurrent use of a polynucleotide 5'-hydroxyl-kinase (e.g., a polynucleotide kinase "PNK") and RtcA or a fusion protein comprising the RtcA amino acid sequence or homologue thereof, and the PAP1 protein or a fusion protein comprising the PAP1 amino acid sequence or a homologue thereof.

Representative examples of fusion protein amino acid sequences are provided above. Use of these enzymes, their RNA substrates with 5' and 3' ends as modified according to a method of this disclosure is depicted in **Fig. 2****.** In an embodiment, the PNK is a T4 PNK, but those skilled in the art will recognize that other PNKs may also be used instead of T4 PNK. Thus, the disclosure comprises use of a PNK or other suitable enzyme to phosphorylate RNA polynucleotides at their 5' ends and dephosphorylate the RNA polynucleotides at their 3' ends, as shown in **Fig. 2**. PNK can be used first, or concurrent with the RtcA, which may be part of a fusion protein that also comprises PAP1. The RtcA catalyzes adenylation of the RNA polynucleotide at their 5'-monophosphate ends. This results in a 5', 5'-adenyl pyrophosphoryl cap structure on the RNA polynucleotides. The PAP1 polyadenylates the 3' end of the RNA polynucleotide.

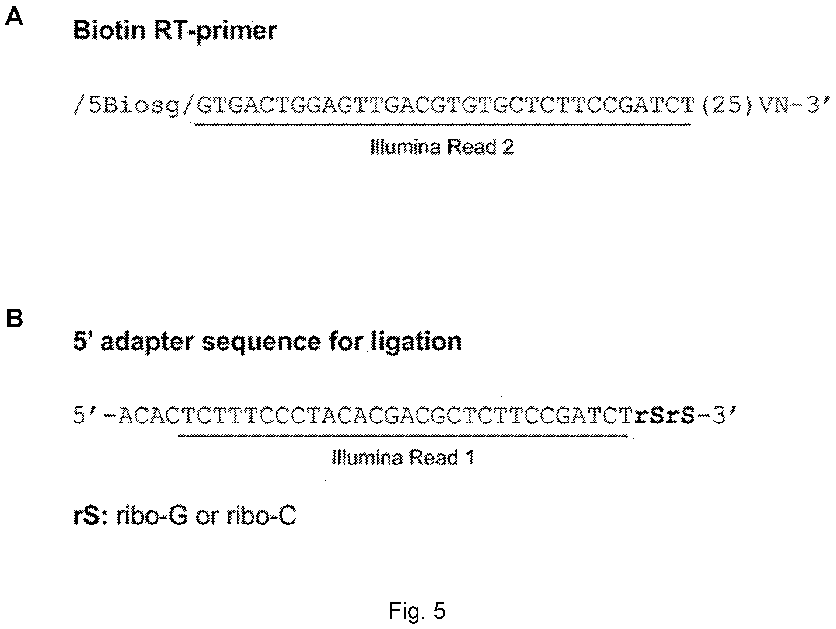

To rapidly enrich the adenylated RNA polynucleotides within a single tube without purification, we designed a poly(dT) oligonucleotide with 5' end biotin labeling (Biotin-RT primer). A representative example of the Biotin-RT primer is shown in **Fig. 5A** and has the following sequence: /5Biosg/GTGACTGGAGTTGACGTGTGCTCTTCCGATCT₍₂₅₎VN (SEQ ID NO:5), wherein V=A, C, or G, and N=A, C, G, or T. After annealing, adenylated RNA polynucleotides together with Biotin-RT primers are precipitated by streptavidin beads. A simple spin down effectively removes adenylation enzymes, which is to facilitate the subsequent 5' end adapter ligation and reverse transcription.

In embodiments, the compositions and methods include an oligonucleotide used as a 5' adapter, and wherein the RNA polynucleotide prepared as described above may be considered a linker. In embodiments, the DNA/RNA hybrid oligonucleotide is shown in **Fig. 5B** and comprises or consists of the following sequence: ACACTCTTTCCCTACACGACGCTCTTCCGATCTrSrS (SEQ ID NO:6), where rS=rG, or rC. In embodiments, the DNA/RNA hybrid oligonucleotides comprise an RNA nucleotide at their 3' ends. In embodiments, the oligonucleotide at the 3' end comprises one or more rSrS-OH (refers to either rCrC-OH or rGrG-OH). In embodiments, a 5' adapter having rSrS at its 3' end is used. In embodiments, an oligonucleotide used in the disclosure does not have rArA at its 3' end. In embodiments, oligonucleotides used in the compositions and methods of the disclosure do not comprise 5' preadenylation, such as for use during conventional cDNA library preparation. In embodiments, the 5' adapters are ligated to the anchored RNA polynucleotides using an RNA ligase, one non-limiting example of which comprises truncated T4 RNA ligase 2 (T4 Rnl2tr).

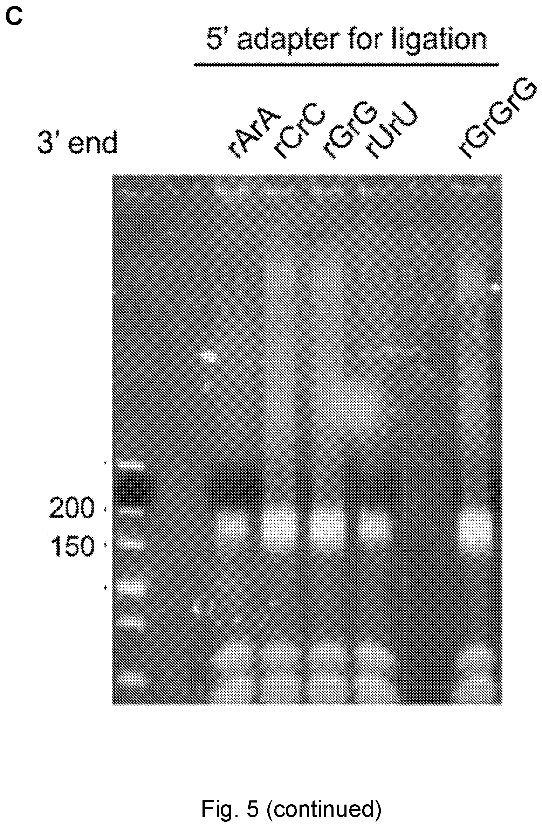

A non-limiting demonstration of ligation efficiency using certain representative oligonucleotides and AppRNA substrates as described above is shown in **Fig. 5C** by way of a photograph of electrophoretic separation of ligated oligonucleotides and RNA adapters prepared as described above. Representative and non-limiting examples of oligonucleotides for use as adapters are shown in **Fig. 5B****.** In embodiments, the oligonucleotides shown in **Fig.** 5B provide an averaged nucleotide length. Accordingly, oligonucleotides with a shorter or longer length can be used.

In specific and non-limiting embodiments, with the 5', 5'-adenyl pyrophosphoryl cap structure, the RNA fragments are converted into a pool of "linkers" which can be ligated to customized RNA adapters with 3'-OH by truncated T4 RNA ligase 2 (T4 Rnl2tr). We designed a DNA/RNA hybrid oligonucleotide ending with varied ribonucleotides at 3' end (rArA, rCrC, rGrG, and rUrU). We found that the truncated T4 RNA ligase 2 (T4 Rnl2tr) efficiently ligated rCrC-OH and rGrG-OH to 5'-AppRNA (**Fig. 5A**). The poor ligation of rArA is important because it prevents self-ligation of polyadenylated AppRNA. Considering the ILLUMINA NextSeq platform, and without intending to be constrained by any particular theory, the 5' adapter ending with rSrS is considered the most suitable for subsequent amplification and sequencing.

In embodiments, the compositions and methods include the cDNA synthesis that directly occurs on the beads (**Fig. 1B**)**.** After removal of non-ligated adapters and T4 Rnl2tr, the cDNA synthesis is achieved by M-MuLV reverse transcriptase.

The final step of PCR reaction is carried out by using common primers complementary to the ILLUMINA sequence elements and bar code sequences. The bar coding system permits pooling of different original samples into one tube, greatly reducing the sequencing cost. During data analysis, the provided bar code information allows rapid separation of original samples. This strategy minimizes technical bias introduced during sequencing. With the clean final products with the correct size (~180 bp), the samples are ready for sequencing.

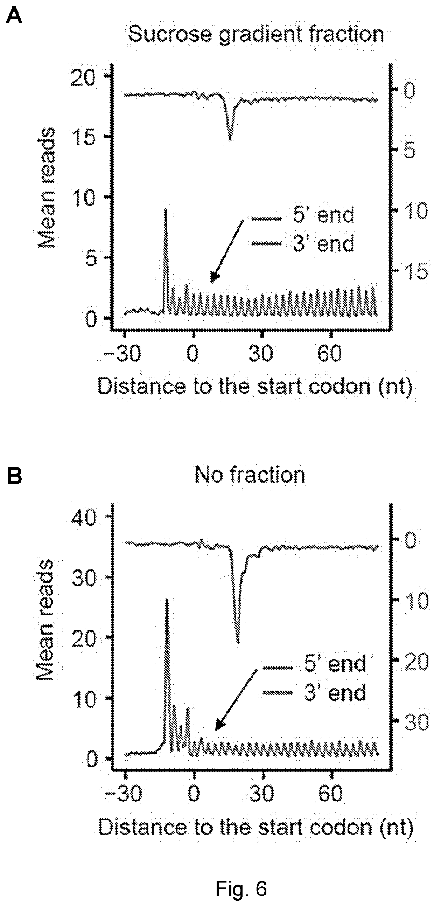

In view of the foregoing, it will be recognized that one application of the compositions and methods described herein is in the Ribo-seq area. In this regard, a hallmark of Ribo-seq is the 3-nt periodicity of RPFs thanks to the relatively precise 5' end protection by elongating ribosomes. As a result, the percentage of reads mapped to the reading frame 0, or in-frame ratio (IFR), has been commonly used to reflect the resolution of Ribo-seq. Optimization of library construction has improved the IFR of RPFs from ~50% to ~75% (**Fig. 1A****,** middle panel vs. left panel) (7). However, prior to the present disclosure, a substantial amount of reads remain out-of-frame, imposing a significant barrier to understanding of ribosome dynamics, especially the reading frame fidelity during translation. The present disclosure addresses these deficiencies and includes additional improvements. Specifically, the presently provided Ezra-seq approach dramatically reduces the amount of starting material (~1 ng RNA), shortens the entire library processing time from 4 days to ~4 hr, and increases the resolution of RPFs with an averaged IFR >90%. As expected from typical Ribo-seq results, Ezra-seq revealed a prominent peaks at start codons, representing the pausing of initiating ribosomes (**Fig. 6A**)**.** It reveals a size of 29-nt of RPFs when both 5' and 3' ends are considered.

With the ultra-resolution of Ezra-seq, ribosome profiling can be achieved without sucrose gradient-based ribosome separation, which has become the bottleneck for its broad application. To test this, we collected whole cell lysates, digested with RNase I, and size-selected 25 - 35 nt RNA species. Remarkably, we obtained the similar results as the one using sucrose gradient ribosome separation (**Fig. 6B**).

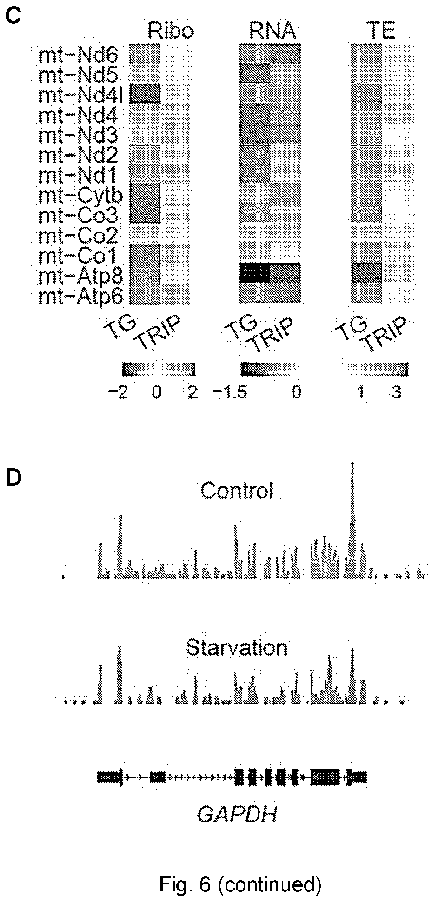

Mitochondria has its own genome and translation machinery. Mitochondrial translation is not as well characterized as that of bacterial and eukaryotic cytoplasmic translation (*11*). From the same original sample, Ezra-seq could capture mitochondrial translation with exquisite sensitivity. We treated mouse embryonic fibroblasts (MEFs) with either thapsigargin (TG, 0.1 µM) or a rhenium compound (TRIP, 25 µM) for 2 hr followed by Ezra-seq. In comparison to the vehicle control, TRIP treatment significantly reduced mitochondrial translation (**Fig. 6C**).

As discussed above, the application of Ezra-seq is not limited to Ribo-seq. Ezra-seq can be readily converted to RNA-seq, serving as a Ribo-seq control in parallel. We applied Ezra-seq to monitor cellular RNA levels and found comparable mitochondria transcripts after TG or TRIP treatment (**Fig. 6C****,** middle panel).

Given the superior sensitivity of Ezra-seq, we also applied Ezra-seq to quantify chromatin-associated RNA species in the nucleus. For many transcripts, Ezra-seq uncovered reads from both intron and exon, an indication of unspliced nascent RNA species (**Fig. 6D**). Interestingly, amino acid starvation for 2 hr resulted in attenuated transcription as exemplified by *GAPDH* (**Fig. 6D**).

The present disclosure also provides articles of manufacture, including but not necessarily limited to kits. In embodiments, the articles of manufacture contain one or more enzymes and/or primers and/or buffers provided in one or more sealed containers, non-limiting examples of which include a sealable glass or plastic vial. The articles of manufacture can include any suitable packaging material, such as a box or envelope or tube to hold the containers. The packaging can include printed material, such as on the packaging or containers themselves, or on a label, or on a paper insert. The printed material can provide a description of using any one of a combination of the enzyme(s), primers and buffer(s) in an assay described herein for the purpose of determining the sequence of any RNA. Any reagent in the article of manufacture/kit can be provided in a form for reconstitution by the user. For example, buffers, primers, enzymes and the like can be provided in dry/power/lyophilized form for making solutions with the reagents. In embodiments, a result based on a determination RNA sequences can be fixed in a tangible medium of expression, such as a digital file saved on a portable memory device, or on a hard drive. This information can be stored, for example, in a digital database for use in a variety of purposes.

The following is an illustrative and non-limiting description of materials and methods that illustrate embodiments of the disclosure. It includes certain specific steps and compositions used in performing Ribo-seq that will be evident from the description, but is otherwise adaptable to sequencing any other plurality of RNA fragments (e.g., fragments of RNA that are not RPFs) by omitting the steps and reagents that pertain to removal of rRNA. The method may be performed using an RNA 3' phosphate cyclase and yeast Poly(A) polymerase separately, or as components of a fusion protein.

### MATERIALS:

### REAGENTS AND BUFFER:

**1. rRNA depletion:**
   1-1. 20×SSC: 3 M NaCl and 0.3 M sodium citrate.
   1-2. 100% ethanol and 70% ethanol.
   1-3. 3 M sodium acetate (pH 5.2).
   1-4. Glycogen (INVITROGEN, AM9510).
**2. For adenylation:**
   2-1. T4 Polynucleotide Kinase.
   2-2. RNA 3'phosphate cyclase
   2-3. Yeast Poly(A) polymerase
   2-4. ATP solution (10 mM) (THERMO FISHER SCIENTIFIC, PV3227).
   2-5. 10×Adenylation buffer: 700 mM Tris-HCl (pH 7.5), 100 mM MgCl₂ and 50 mM DTT.
   2-6. SUPERase_In RNase Inhibitor (INVITROGEN, AM2696).
**3. For beads binding:**
   3-1. Streptavidin Magnetic Beads (NEW ENGLAND BIOLABS, S1420S)
**4. For oligo ligation:**
   4-1. T4 RNA Ligase 2, truncated, K227Q (NEW ENGLAND BIOLABS, M0351L), with PEG8000 50% (w/v) and 10× T4 RNA ligase buffer.
**5. For cDNA synthesis:**
   5-1. 5× first strand buffer: 250 mM Tris-HCl (pH 8.3), 375 mM KCl and 15 mM MgCl₂.
   5-2. 0.1 M DTT.
   5-3. M-MuLV reverse transcriptase mut5 (homemade).
   5-4. RNaseOUT (Invitrogen, 10777-019).
**6. For PCR reaction:**
   6-1. Phusion HF Buffer (THERMO FISHER SCIENTIFIC, F518L)
   6-2. dNTP mix (10mM) (THERMO FISHER SCIENTIFIC, 18427013)
   6-3. Phusion DNA Polymerase (homemade).
**7. For size selection:** (for Ribo-seq only)
   7-1. Novex Hi-Density TBE Sample Buffer (5X) (INVITROGEN, LC6678).
   7-2. Novex 5× TBE running buffer (INVITROGEN, LC6675).
   7-3. DNA gel extraction buffer: 10 mM Tris (pH 8.0), 300 mM NaCl and 1 mM EDTA.
   7-4. SYBR Gold nucleic acid gel stain (INVITROGEN, S-11494).

### EQUIPMENT:

**1. For library construction:**
   1-1. DNA LoBind Tube 1.5 ml (EPPENDORF, 022431021).
   1-2. Heat block.
   1-3. Refrigerated micro centrifuge.
   1-4. Magnetic separation rack.
   1-5. PCR tube with lid.
**2. For gel running and size selection:**
   2-1. Electrophoresis power supply.
   2-2. Mini-Cell polyacrylamide gel box (THERMO FISHER SCIENTIFIC, EI0001).
   2-3. Novex TBE Gels, 8% (Invitrogen, EC6215BOX).
   2-4. Blue light illuminator.
   2-5. Razors.
   2-6. Spin-X centrifuge tube filters, 0.22µm Pore CA Membrane (SIGMA, CLS8160).

### OLIGOS AND PRIMERS:

**1. rRNA depletion (de-rRNA) oligos: (5'→3')**
   /Biotin-TEG/AGCGAGCGACCAAAGGAACCATAACTGATTT (SEQ ID NO:7)
   /Biotin-TEG/CGAGGTTATCTAGAGTCACCA (SEQ ID NO:8)
   /Biotin-TEG/TCCTAGCTGCGGTATCCAGGCG (SEQ ID NO:9)
   /Biotin-TEG/AGATAGTCAAGTTCGACCGTCTTCTCAGC (SEQ ID NO:10)
   /Biotin-TEG/GGGCCTCGATCAGAAGGACTTGGGCCCCCCACGA (SEQ ID NO:11)
   /Biotin-TEG/GCGAGACGGGCCGGTGGTGCGCCCTCGGCGG (SEQ ID NO:12)
   /Biotin-TEG/CGCTTGGCGCCAGAAGCGAGAGCC (SEQ ID NO:13)
   /Biotin-TEG/AGCGACGCTCAGACAGGCGTAGCCCCGGGAG (SEQ ID NO:14)
   /Biotin-TEG/CCGGCTATCCGAGGCCAACCG (SEQ ID NO:15)
   /Biotin-TEG/TGATCTGATAAATGCACGCATCCCCC (SEQ ID NO:16)
   /Biotin-TEG/GTGTCGAGGGCTGACTTTCAATAGATCGCAGCG (SEQ ID NO:17)
   /Biotin-TEG/AGTAGTGGTATTTCACCGGCG (SEQ ID NO:18)
   /Biotin-TEG/TAGAATTACCACAGTTATC (SEQ ID NO:19)
**2. Ligation Oligo mix:(5'→3')**
   ACACTCTTTCCCTACACGACGCTCTTCCGATCTrSrS (SEQ ID NO:6) rS: ribo-G or C
**3.Biotin RT-primer:(5'→3')**
   /5Biosg/GTGACTGGAGTTGACGTGTGCTCTTCCGATCT₍₂₅₎VN (SEQ ID NO:5)
**4.PCR primers:(5'→3')** 
F:
AATGATACGGCGACCACCGAGATCTACACTCTTTCCCTACACGACGCTCTTCCGA
TCT (SEQ ID NO:20)
 
R: CAAGCAGAAGACGGCATACGAGAT NNNNNN
GTGACTGGAGTTCAGACGTGTGCTCTTCCGATCT (SEQ ID NO:21)
 ** NNNNNN:* barcode (index)

### PROCEDURES:

**Starting Material:** RNA fragments (10~200 ng) in 10 µL Nuclease-Free H₂O.
**1. rRNA depletion (Optional, Timing: 80 min):**
1-1. Prepare rRNA depletion master mix as in Table 1:

**Table A**

| Components | Amount |
|---|---|
| 25 µM de-rRNA Oligo mix | 1.0 µL |
| 20× SSC | 2.0 µL |
| Nuclease-Free water | 7.0 µL |
| Total | 10.0 µL |

1-2. Add 10 µL rRNA depletion master mix to RNA sample (20 µL total)
1-3. Incubate at 80°C for 30s, followed by slow cooling (~3°C/min) to 37°C.
1-4. Pre-wash streptavidin magnetic beads as below during incubation:
1-4-1. Add suspended streptavidin magnetic beads (20 µL/sample) into a new 1.5mL tube.
1-4-2. Place the tube into a magnetic stand for 1-2 min. Remove and discard the supernatant.
1-4-3. Add 200 µL of 2× SSC to the tube, vortex gently to mix. Collect the beads with a magnetic stand, then remove and discard the supernatant.
1-4-4. Suspend streptavidin magnetic beads (20 µL/sample) in 2× SSC.

1-5. Add 20 µL streptavidin beads (pre-washed), mix well by pipetting several times, keep at room temperature for 10 min.
1-6. Place tube on magnet for 2 min, then transfer supernatant (40 µL total) into a new 1.5mL tube, discard the beads.
1-7. Precipitate RNA sample:
1-7-1. Add 160 µL Nuclease-Free water, 4 µL glycogen and 40 µL 3M sodium acetate, and then 500 µL 100% ethanol.
1-7-2. Precipitate for at least 30 min at -20°C.
1-7-3. Pellet the RNA by centrifugation for 15 min at 20,000g, 4°C.
1-7-4. Wash RNA pellet with 500 µL 70% ethanol, followed by centrifugation for 5 min at 20,000g, 4°C, pipette all liquid from the tube and air-dry for 5 min.

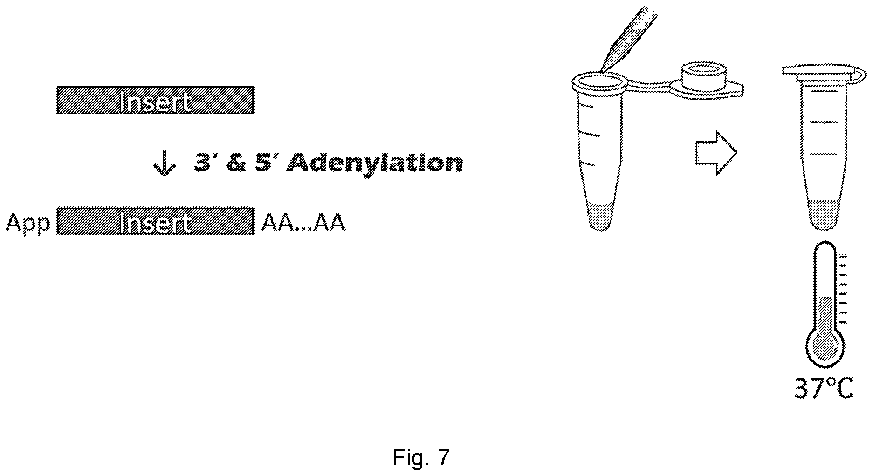

1-8. Dissolve RNA pallet in 10 µL Nuclease-Free H₂O.
**2. 3' & 5'-Adenylation (Timing: 30 min)** (depicted in Figure 7), wherein the "insert" the RNA segment to be sequenced, and for Ribo-seq, it is ribosome-protected fragments (RPF)).
2-1. Prepare adenylation master mix as shown in Table B:

**Table B**

| Components | Amount |
|---|---|
| 10× Adenylation buffer | 2.0 µL |
| 10 mM ATP | 2.0 µL |
| 20 U/µL SUPERase_In | 1.0 µL |
| 125 µg/mL Yeast poly(A) polymerase | 1.0 µL |
| 50 µg/mL RtcA | 1.0 µL |
| 250 µg/mL T4 polynucleotide kinase | 1.0 µL |
| Total | 8.0 µL |

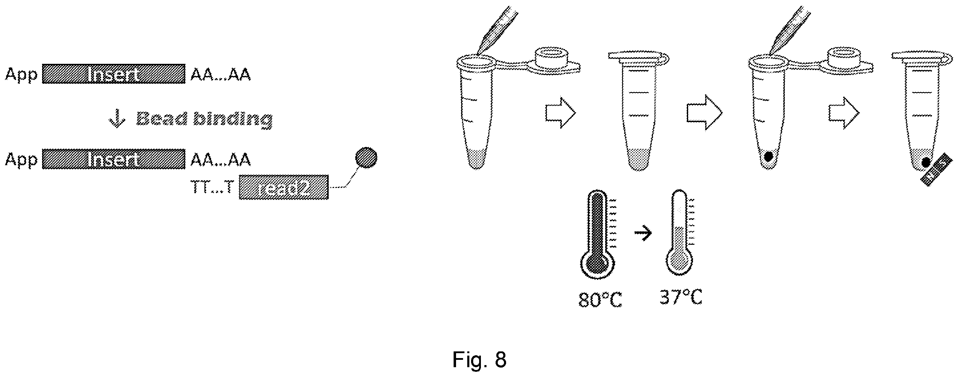

2-2. Add 8.0 µL adenylation master mix to sample tube (20 µL total).
2-3. Mix and incubate at 37°C for 30 min.
**3. Bead binding (Timing: 30 min):** (depicted in Figure 8)
3-1. Prepare beads binding master mix as described in Table C:

**Table C**

| Components | Amount |
|---|---|
| 20× SSC | 4.0 µL |
| 10 µM Biotin-RT primer | 1.0 µL |
| Nuclease free H₂O | 15.0 µL |
| Total | 20.0 µL |

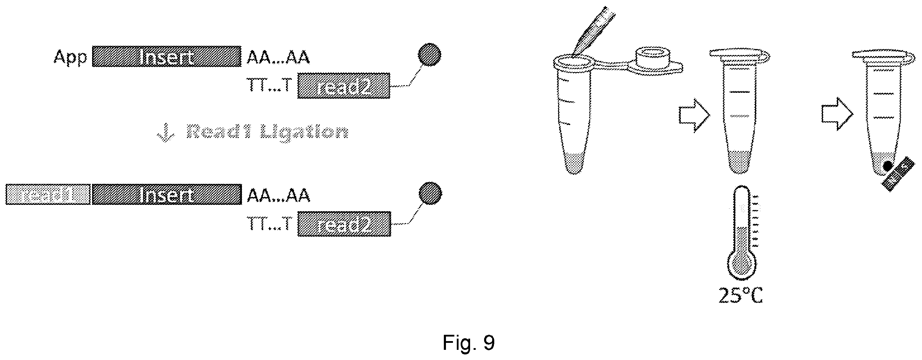

3-2. Add 20 µL binding master mix to sample tube (40 µL total).
3-3. Mix and incubate at 65°C for 3 min, then cool down (~3°C/min) to room temperature.
3-4. Pre-wash streptavidin magnetic beads (10 µL/sample) as described at step 1-4 during incubation.
3-5. Add 10 µL pre-washed streptavidin beads to each tube, mix by pipetting several times.
3-6. Incubate at room temperature for 10 min.
3-7. Place tube on magnet for 1-2 min to collect the beads and remove the supernatant.
3-8. Re-suspend beads in 200 µL Nuclease free H₂O.
3-9. Place tube on magnet for 1-2 min and remove the supernatant.
**4. Ligation [Timing: 125 min]** (depicted in Figure 9, wherein "read1" and "read2" are primer sequences adapted for use in, for example, ILLUMINA sequencing)
4-1. Re-suspend the beads with 9 µL Nuclease-Free H₂O and 1µl of 10 µM Ligation oligos by pipetting several times.
4-2. Prepare ligation master mix as described in Table D:

**Table D**

| Components | Amount |
|---|---|
| 10x T4 RNA ligase buffer | 2.0µL |
| 50% PEG8000 | 6.0µL |
| 20 U/µL SUPERase_In | 1.0µL |
| 200 U/µL T4 RNL2 Ligase | 1.0µL |
| Total | 10.0µL |

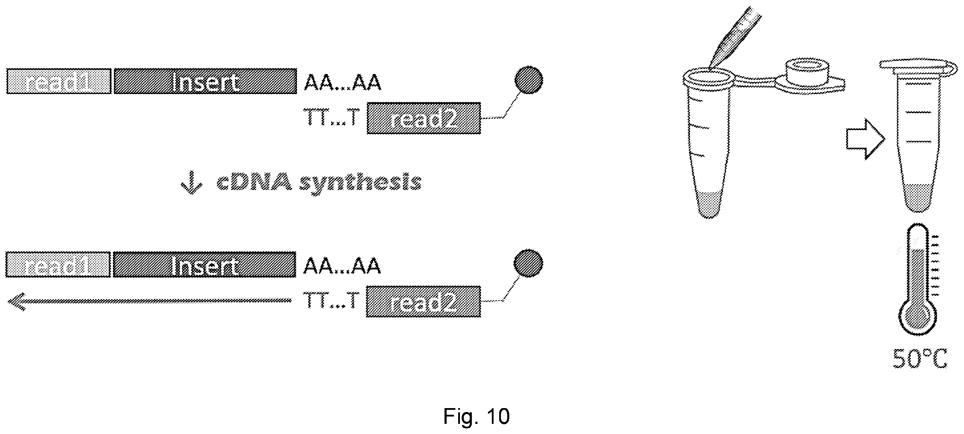

4-3. Add the 10 µL ligation master mix to sample tube (20 µL total volume).
4-4. Mix and incubate at 25°C (or room temperature) for 120 min with continuous shaking at 800 rpm.
4-5. Place tube on magnet for 1-2 min to collect the beads and remove the supernatant.
4-6. Re-suspend beads in 200 µL Nuclease free H₂O.
4-7. Place tube on magnet for 1-2 min and remove the all the supernatant.
**5. cDNA synthesis [Timing: 35 min]** (depicted in Figure 10)
5-1. Re-suspend the beads with 13 µL Nuclease-Free H₂O by pipetting several times.
5-2. Incubate at 70°C for 2 min, then let sample cool to room temperature.
5-3. Prepare cDNA synthesis master mix as described in Table E:

**Table E**

| Components | Amount |
|---|---|
| 5× first strand buffer | 4.0µL |
| 0.1 M DTT | 1.0µL |
| 10 mM dNTP | 0.5µL |
| 40 U/µL RNaseOUT | 0.5µL |
| 500 µg/mL m-MLV mut-5 | 1.0µL |
| Total | 7.0 µL |

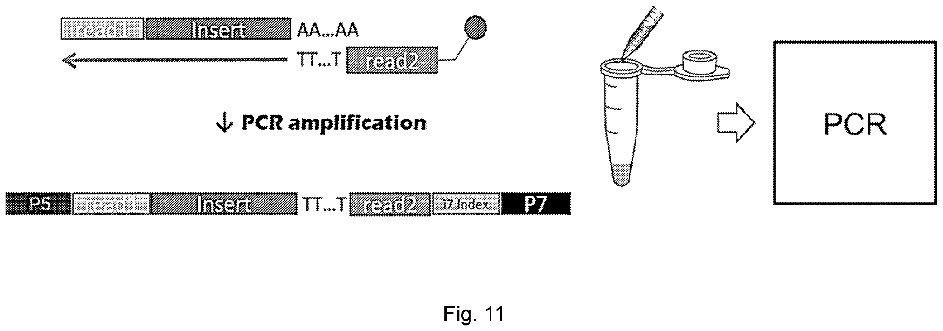

5-4. Add 7 µL cDNA synthesis master mix to sample tube (20 µL total).
5-5. Mix well and incubate at 50°C for 30 min with continuous shaking at 800 rpm, then 85°C for 10 min to terminate.
**6. PCR amplification [Timing: 30 min]** (depicted in Figure 11, wherein P5 and P7 are ILLUMINA sequences used for cluster formation, and the i7 index a barcode sequence).
6-1. Prepare PCR master mix as described in Table F:

**Table F**

| Components | Amount |
|---|---|
| 5*HF buffer | 4.0 µL |
| Nuclease-free H₂O | 12.75 µL |
| 10 mM dNTP | 1.0 µL |
| 10 µM Forward Primer | 0.5 µL |
| 10 µM Reverse Primer (with barcode) | 0.5 µL |
| 2 U/µL Phusion^{®} High-Fidelity DNA Polymerase | 0.25 µL |
| Total | 7.0 µL |

6-2. Add the 2 µL cDNA from step 5 and 18 µL PCR master mix (20 µL total) into PCR tubes.
Different PCR primers with distinct barcodes are used for each sample to be pooled.
6-3. Perform PCR reaction as described below in Table G:

**Table G:**

| Cycle number | Denature | Anneal | Extend |
|---|---|---|---|
| 1 | 98°C, 30s | 67°C, 15s | 72°C, 10s |
| 10-14 | 98°C, 5s | | |
| 1 | 72°C, 3min | | |

**7. Size selection (Timing: 50 min)** (only applicable to Ribo-seq):
7-1. Add 5 µL Novex^{™} Hi-Density TBE Sample Buffer (5X) to each PCR tube.
7-2. Load sample on an 8% Novex^{™} TBE Gels and run electrophoresis at 180V for 45min.
7-3. Stain the gel in 1×SYBR Gold for 3-5 min.
7-4. Visualize the gel and excise the target PCR product.
7-5. Recover PCR product in 400 µL DNA gel extraction buffer at 4°C overnight on with rotation.

**8. QC and sequencing** (only applicable to Ribo-seq):
8-1. Precipitate DNA as described at step 1-7.
8-2. Dissolve the RNA pallet in 15 µL Nuclease-Free H₂O.
8-3. Send for QC and sequencing.

### References

1. X. Adiconis et al., Nat Methods 15, 505 (2018).
2. N. T. Ingolia, S. Ghaemmaghami, J. R. Newman, J. S. Weissman, Science 324, 218 (2009).
3. M. V. Gerashchenko, V. N. Gladyshev, Nucleic Acids Res 42, e134 (2014).
4. D. A. Santos, L. Shi, B. P. Tu, J. S. Weissman, Nucleic Acids Res 47, 4974 (2019).
5. N. Hornstein et al., Genome Biol 17, 149 (2016).
6. A. Turchinovich et al., RNA Biol 11, 817 (2014).
7. N. T. Ingolia, G. A. Brar, S. Rouskin, A. M. McGeachy, J. S. Weissman, Nat Protoc 7, 1534 (2012).
8. L. Lama, J. Cobo, D. Buenaventura, K. Ryan, J Biol Methods 6, (2019).
9. Y. Wang, S. K. Silverman, RNA 12, 1142 (2006).
10. A. K. Chakravarty, S. Shuman, J Biol Chem 286, 4117 (2011).
11. B. E. Christian, L. L. Spremulli, Biochim Biophys Acta 1819, 1035 (2012).

## Claims

1. A method for determining nucleotide sequences of RNA polynucleotides, the method comprising:
a) providing a plurality of RNA fragments obtained from the RNA polynucleotides;
b) enzymatically phosphorylating 5' ends of the plurality of RNA fragments to provide a plurality of RNA fragments comprising mono-phosphorylated 5' ends;
c) enzymatically dephosphorylating 3' ends of the plurality of RNA fragments to provide a plurality of RNA fragments comprising free 3' hydroxyls;
wherein a ligase enzymatically phosphorylates the 5' ends of the plurality of RNA fragments to provide a plurality of RNA fragments comprising mono-phosphorylated 5' ends, and wherein the ligase enzymatically dephosphorylates 3' ends of the plurality of RNA fragments to provide the plurality of RNA fragments comprising the free 3' hydroxyls;
d) enzymatically adenylylating phosphorylated 5' ends of the plurality of RNA fragments to provide a plurality of 5' mono-adenylated RNA fragments;
e) enzymatically polyadenylating 3' ends of the plurality of RNA fragments comprising the free 3' hydroxyls to provide a plurality of RNA fragments comprising polyadenylated 3' ends;
wherein the enzymatically adenylating the phosphorylated 5' ends of the plurality of RNA fragments, and the polyadenylating of the 3' ends of the plurality of RNA fragments, is performed using a cyclase and a polymerase, wherein the cyclase comprises an RtcA enzyme;
f) ligating oligonucleotide adapters to the 5' ends of the plurality of 5' mono-adenylated RNA fragments, the oligonucleotide adapters optionally comprising a DNA/RNA hybrid 3' end, the DNA/RNA hybrid 3' end optionally comprising rCrC-OH or rGrG-OH as the RNA component of the DNA/RNA hybrid, to provide a plurality of RNA fragments comprising the oligonucleotide adapters at the 5' ends;
g) generating cDNAs from the plurality of RNA fragments of f);
h) amplifying the cDNAs; and
i) determining nucleotide sequences of the cDNAs, thereby determining the nucleotide sequences of the RNA polynucleotides.

2. The method of claim 1, wherein at least b)-h) is performed in a single reaction container.

3. The method of claim 2, wherein the reaction container comprises a substrate that optionally comprises streptavidin, and wherein a primer used to generate the cDNAs by reverse transcription comprises a binding partner that binds to the substrate, the binding partner optionally comprising biotin.

4. The method of claim 2, wherein at least 80% of the 5' ends of the plurality of RNA fragments are sequenced.

5. The method of claim 2, wherein the plurality of RNA polynucleotide fragments comprise open reading frames, and wherein an in-frame ratio (IFR) of at least 90% is obtained for sequenced RNA polynucleotides.

6. The method of any one of claims 1-5, wherein the ligase is T4 RNA ligase 2 (T4 Rnl2tr).

7. The method of claim 6, wherein the cyclase and the polymerase are configured as a single fusion protein;
wherein, optionally, the polymerase comprises poly(A) polymerase obtained or derived from *E. coli* poly(A) polymerase (*E. coli* PAP1) or *Saccharomyces cerevisiae* poly(A) polymerase (*S. cerevisiae* PAP1).

8. The method of any one of claims 1-5, wherein the ligating the oligonucleotide adapters to the 5' ends of the plurality of the 5' mono-adenylated RNA fragments is optionally performed using a T4 RNA ligase.

9. A composition comprising a mixture of two distinct proteins, or a fusion protein, for use in RNA sequencing, the two distinct proteins or the fusion protein comprising a poly(A) polymerase and an RNA 3'-phosphate cyclase, wherein the cyclase comprises an RtcA enzyme;
optionally comprising the fusion protein.

10. An isolated fusion protein comprising a poly(A) polymerase and an RNA 3'-phosphate cyclase, wherein the cyclase comprises an RtcA enzyme.

11. A kit comprising a mixture of two distinct proteins or a fusion protein for use in RNA sequencing, the two distinct proteins or the fusion protein comprising a poly(A) polymerase and an RNA 3'-phosphate cyclase comprising an RtcA enzyme; the kit further optionally comprising at least one of an RNA ligase or an RNA kinase, the kit further comprising at least one container that contains at least the mixture of the two distinct proteins or the fusion protein.

12. The kit of claim 11, further comprising at least one oligonucleotide primer for use in cDNA synthesis, wherein the oligonucleotide primer comprises a poly-T segment, and wherein the oligonucleotide primer is optionally labeled such that it can be bound to a binding partner, wherein said label optionally comprises biotin;
optionally further comprising a plurality of beads comprising a moiety configured to bind to the label, wherein the moiety optionally comprises streptavidin, and wherein the plurality of beads optionally comprise magnetic beads; and / or
comprising at least one buffer, said buffer having a pH of approximately 7.0, and/or an ATP concentration that is greater than 1 mM, and is optionally approximately 2 mM.

13. An article of manufacture comprising at least one sealed container, the at least one sealed container containing at least the mixture of two distinct proteins or the fusion protein of claim 9, the article of manufacture further comprising printed material that provides an indication that contents of the article of manufacture are for use in RNA sequencing.

14. The article of manufacture of claim 13, further comprising one or a combination of:
i) at least one oligonucleotide primer for use in cDNA synthesis, wherein the oligonucleotide primer comprises a poly-T segment, and wherein the oligonucleotide primer is optionally labeled such that it can be bound to a binding partner, wherein said label optionally comprises biotin;
ii) a plurality of beads comprising a moiety configured to bind to the label, wherein the moiety optionally comprises streptavidin, and wherein the plurality of beads optionally comprise magnetic beads.

15. The article of manufacture of claim 14, further comprising at least one sealed container that comprises a buffer or components to make the buffer, the buffer having a pH of approximately 7.0, and/or an ATP concentration that is greater than 1 mM, and is optionally approximately a 2 mM concentration of the ATP.

## Patentansprüche

1. Verfahren zum Bestimmen von Nukleotidsequenzen von RNA-Polynukleotiden, wobei das Verfahren Folgendes umfasst:
a) Bereitstellen einer Vielzahl von RNA-Fragmenten, die aus den RNA-Polynukleotiden erlangt wurden;
b) enzymatisches Phosphorylieren von 5'-Enden der Vielzahl von RNA-Fragmenten, um eine Vielzahl von RNA-Fragmenten, umfassend monophosphorylierte 5'-Enden, bereitzustellen;
c) enzymatisches Dephosphorylieren von 3'-Enden der Vielzahl von RNA-Fragmenten, um eine Vielzahl von RNA-Fragmenten, umfassend freie 3'-Hydroxyle, bereitzustellen;
wobei eine Ligase enzymatisch die 5'-Enden der Vielzahl von RNA-Fragmenten phosphoryliert, um eine Vielzahl von RNA-Fragmenten, umfassend monophosphorylierte 5'-Enden, bereitzustellen, und wobei die Ligase enzymatisch 3'-Enden der Vielzahl von RNA-Fragmenten dephosphoryliert, um die Vielzahl von RNA-Fragmenten, umfassend die freien 3'-Hydroxyle, bereitzustellen;
d) enzymatisches Adenylieren von phosphorylierten 5'-Enden der Vielzahl von RNA-Fragmenten, um eine Vielzahl von 5'-monoadenylierten RNA-Fragmenten bereitzustellen;
e) enzymatisches Polyadenylieren von 3'-Enden der Vielzahl von RNA-Fragmenten, umfassend die freien 3'-Hydroxyle, um eine Vielzahl von RNA-Fragmenten, umfassend polyadenylierte 3'-Enden, bereitzustellen;
wobei das enzymatische Adenylieren der phosphorylierten 5'-Enden der Vielzahl von RNA-Fragmenten und das Polyadenylieren der 3'-Enden der Vielzahl von RNA-Fragmenten mithilfe einer Cyclase und einer Polymerase durchgeführt wird, wobei die Cyclase ein RtcA-Enzym umfasst;
f) Ligieren von Oligonukleotidadaptern an die 5'-Enden der Vielzahl von 5'-monoadenylierten RNA-Fragmenten, wobei die Oligonukleotidadapter optional ein DNA/RNA-Hybrid-3'-Ende umfassen, wobei das DNA/RNA-Hybrid-3'-Ende optional rCrC-OH oder rGrG-OH als die RNA-Komponente des DNA/RNA-Hybrids umfasst, um eine Vielzahl von RNA-Fragmenten, umfassend die Oligonukleotidadapter an den 5'-Enden, bereitzustellen;
g) Erzeugen von cDNAs aus der Vielzahl von RNA-Fragmenten von f);
h) Amplifizieren der cDNAs; und
i) Bestimmen der Nukleotidsequenzen der cDNAs, wodurch die Nukleotidsequenzen der RNA-Polynukleotide bestimmt werden.

2. Verfahren nach Anspruch 1, wobei mindestens b) bis h) in einem einzigen Umsetzungsbehälter durchgeführt werden.

3. Verfahren nach Anspruch 2, wobei der Umsetzungsbehälter ein Substrat umfasst, das optional Streptavidin umfasst, und wobei ein Primer, der verwendet wird, um die cDNAs durch reverse Transkription zu erzeugen, einen Bindungspartner umfasst, der sich an das Substrat bindet, wobei der Bindungspartner optional Biotin umfasst.

4. Verfahren nach Anspruch 2, wobei mindestens 80 % der 5'-Enden der Vielzahl von RNA-Fragmenten sequenziert werden.

5. Verfahren nach Anspruch 2, wobei die Vielzahl von RNA-Polynukleotidfragmenten offene Leserahmen umfassen und wobei ein In-Frame-Verhältnis (IFR) von mindestens 90 % für sequenzierte RNA-Polynukleotide erlangt wird.

6. Verfahren nach einem der Ansprüche 1 bis 5, wobei die Ligase eine T4-RNA-Ligase 2 (T4 Rnl2tr) ist.

7. Verfahren nach Anspruch 6, wobei die Cyclase und die Polymerase als ein einziges Fusionsprotein konfiguriert sind; wobei die Polymerase optional Poly(A)-Polymerase umfasst, die aus E.-coli-Poly(A)-Polymerase *(E. coli* PAP1) oder *Saccharomyces-cerevisiae-Poly(A)-Polymerase* (*S*. *cerevisiae* PAP1) erlangt wird oder davon abgeleitet ist.

8. Verfahren nach einem der Ansprüche 1 bis 5, wobei das Ligieren der Oligonukleotidadapter an die 5'-Enden der Vielzahl von 5'-monoadenylierten RNA-Fragmenten optional mithilfe einer T4-RNA-Ligase durchgeführt wird.

9. Zusammensetzung, umfassend ein Gemisch aus zwei verschiedenen Proteinen oder ein Fusionsprotein zur Verwendung bei einem RNA-Sequenzieren, wobei die zwei verschiedenen Proteine oder das Fusionsprotein eine Poly(A)- Polymerase und eine RNA-3'-Phosphat-Cyclase umfassen, wobei die Cyclase ein RtcA-Enzym umfasst;
optional umfassend das Fusionsprotein.

10. Isoliertes Fusionsprotein, umfassend eine Poly(A)-Polymerase und eine RNA-3'-Phosphat-Cyclase, wobei die Cyclase ein RtcA-Enzym umfasst.

11. Satz, umfassend ein Gemisch aus zwei verschiedenen Proteinen oder ein Fusionsprotein zur Verwendung bei einem RNA-Sequenzieren, wobei die zwei verschiedenen Proteine oder das Fusionsprotein eine Poly(A)-Polymerase und eine RNA-3'-Phosphat-Cyclase, umfassend ein RtcA-Enzym, umfassen; wobei der Satz ferner optional mindestens eines von einer RNA-Ligase oder einer RNA-Kinase umfasst, wobei der Satz ferner mindestens einen Behälter umfasst, der mindestens das Gemisch aus den zwei verschiedenen Proteinen oder das Fusionsprotein enthält.

12. Satz nach Anspruch 11, ferner umfassend mindestens einen Oligonukleotidprimer zur Verwendung bei einer cDNA-Synthese, wobei der Oligonukleotidprimer ein Poly-T-Segment umfasst und wobei der Oligonukleotidprimer optional derart markiert ist, dass er an einen Bindungspartner gebunden werden kann, wobei die Markierung optional Biotin umfasst;
optional ferner umfassend eine Vielzahl von Perlen, umfassend eine Einheit, die konfiguriert ist, um sich an die Markierung zu binden, wobei die Einheit optional Streptavidin umfasst und wobei die Vielzahl von Perlen optional magnetische Perlen umfasst; und/oder
umfassend mindestens einen Puffer, wobei der Puffer einen pH-Wert von etwa 7,0 und/oder eine ATP-Konzentration aufweist, die größer als 1 mM ist und optional etwa 2 mM ist.

13. Gegenstand zur Herstellung, umfassend mindestens einen versiegelten Behälter, wobei der mindestens eine versiegelte Behälter mindestens das Gemisch aus zwei verschiedenen Proteinen oder das Fusionsprotein nach Anspruch 9 enthält, wobei der Gegenstand zur Herstellung ferner bedrucktes Material umfasst, das eine Anzeige bereitstellt, dass Inhalte des Gegenstands zur Herstellung zur Verwendung bei einem RNA-Sequenzieren sind.

14. Gegenstand zur Herstellung nach Anspruch 13, ferner umfassend eines oder eine Kombination von Folgendem:
i) mindestens einem Oligonukleotidprimer zur Verwendung bei einer cDNA-Synthese, wobei der Oligonukleotidprimer ein Poly-T-Segment umfasst und wobei der Oligonukleotidprimer optional derart markiert ist, dass er an einen Bindungspartner gebunden werden kann, wobei die Markierung optional Biotin umfasst;
ii) einer Vielzahl von Perlen, umfassend eine Einheit, die konfiguriert ist, um sich an die Markierung zu binden, wobei die Einheit optional Streptavidin umfasst und wobei die Vielzahl von Perlen optional magnetische Perlen umfasst.

15. Gegenstand zur Herstellung nach Anspruch 14, ferner umfassend mindestens einen versiegelten Behälter, der einen Puffer oder Komponenten enthält, um den Puffer herzustellen, wobei der Puffer einen pH-Wert von etwa 7,0 und/oder eine ATP-Konzentration aufweist, die größer als 1 mM ist und optional etwa eine 2-mM-Konzentration von ATP ist.

## Revendications

1. Procédé de détermination de séquences nucléotidiques de polynucléotides d'ARN, le procédé comprenant :
a) la fourniture d'une pluralité de fragments d'ARN obtenus à partir des polynucléotides d'ARN ;
b) la phosphorylation par voie enzymatique des extrémités 5' de la pluralité de fragments d'ARN pour fournir une pluralité de fragments d'ARN comprenant des extrémités 5' monophosphorylées ;
c) la déphosphorylation par voie enzymatique des extrémités 3' de la pluralité de fragments d'ARN pour fournir une pluralité de fragments d'ARN comprenant des hydroxyles 3' libres ;
une ligase phosphorylant enzymatiquement les extrémités 5' de la pluralité de fragments d'ARN pour fournir une pluralité de fragments d'ARN comprenant des extrémités 5' monophosphorylées, et la ligase déphosphorylant enzymatiquement les extrémités 3' de la pluralité de fragments d'ARN pour fournir la pluralité de fragments d'ARN comprenant les hydroxyles 3' libres ;
d) l'adénylation par voie enzymatique des extrémités 5' phosphorylées de la pluralité de fragments d'ARN pour fournir une pluralité de fragments d'ARN mono-adénylés en 5' ;
e) la polyadénylation par voie enzymatique des extrémités 3' de la pluralité de fragments d'ARN comprenant les hydroxyles 3' libres pour fournir une pluralité de fragments d'ARN comprenant des extrémités 3' polyadénylées ;
l'adénylation par voie enzymatique des extrémités 5' phosphorylées de la pluralité de fragments d'ARN, et la polyadénylation des extrémités 3' de la pluralité de fragments d'ARN, étant réalisées à l'aide d'une cyclase et d'une polymérase, la cyclase comprenant une enzyme RtcA ;
f) la ligature d'adaptateurs oligonucléotidiques aux extrémités 5' de la pluralité de fragments d'ARN mono-adénylés en 5', les adaptateurs oligonucléotidiques comprenant éventuellement une extrémité 3' hybride ADN/ARN, l'extrémité 3' hybride ADN/ARN comprenant éventuellement rCrC-OH ou rGrG-OH comme composant ARN de l'hybride ADN/ARN, pour fournir une pluralité de fragments d'ARN comprenant les adaptateurs oligonucléotidiques aux extrémités 5' ;
g) la génération d'ADNc à partir de la pluralité de fragments d'ARN de f) ;
h) l'amplification des ADNc ; et
i) la détermination des séquences nucléotidiques des ADNc, déterminant ainsi les séquences nucléotidiques des polynucléotides d'ARN.

2. Procédé selon la revendication 1, dans lequel au moins les étapes b) à h) sont réalisées dans un seul récipient de réaction.

3. Procédé selon la revendication 2, dans lequel le récipient de réaction comprend un substrat qui comprend éventuellement de la streptavidine, et dans lequel une amorce utilisée pour générer les ADNc par transcription inverse comprend un partenaire de liaison qui se lie au substrat, le partenaire de liaison comprenant éventuellement de la biotine.

4. Procédé selon la revendication 2, dans lequel au moins 80 % des extrémités 5' de la pluralité de fragments d'ARN sont séquencées.

5. Procédé selon la revendication 2, dans lequel la pluralité de fragments de polynucléotides d'ARN comprennent des cadres de lecture ouverts, et dans lequel un rapport dans le cadre (IFR) d'au moins 90 % est obtenu pour les polynucléotides d'ARN séquencés.

6. Procédé selon l'une quelconque des revendications 1 à 5, dans lequel la ligase est la ligase ARN T4 2 (T4 Rnl2tr).

7. Procédé selon la revendication 6, dans lequel la cyclase et la polymérase sont configurées comme une seule protéine de fusion ;
dans lequel, éventuellement, la polymérase comprend une poly(A) polymérase obtenue ou dérivée de la poly(A) polymérase *d'E. coli* (*E. coli* PAP1) ou de la poly(A) polymérase *de Saccharomyces cerevisiae* (*S. cerevisiae* PAP1).

8. Procédé selon l'une quelconque des revendications 1 à 5, dans lequel la ligature des adaptateurs oligonucléotidiques aux extrémités 5' de la pluralité des fragments d'ARN mono-adénylés en 5' est éventuellement réalisée à l'aide d'une ligase ARN T4.

9. Composition comprenant un mélange de deux protéines distinctes, ou une protéine de fusion, destinée à être utilisée dans le séquençage d'ARN, les deux protéines distinctes ou la protéine de fusion comprenant une poly(A) polymérase et une cyclase à 3'-phosphate d'ARN, la cyclase comprenant une enzyme RtcA ;
comprenant éventuellement la protéine de fusion.

10. Protéine de fusion isolée comprenant une poly(A) polymérase et une cyclase à 3'-phosphate d'ARN, la cyclase comprenant une enzyme RtcA.

11. Kit comprenant un mélange de deux protéines distinctes ou une protéine de fusion destinée (s) à être utilisée(s) dans le séquençage d'ARN, les deux protéines distinctes ou la protéine de fusion comprenant une poly(A) polymérase et une cyclase à 3'-phosphate d'ARN comprenant une enzyme RtcA ; le kit comprenant en outre éventuellement au moins une ligase d'ARN ou une kinase d'ARN, le kit comprenant en outre au moins un récipient qui contient au moins le mélange des deux protéines distinctes ou la protéine de fusion.

12. Kit selon la revendication 11, comprenant en outre au moins une amorce oligonucléotidique destinée à être utilisée dans la synthèse d'ADNc, l'amorce oligonucléotidique comprenant un segment poly-T, et l'amorce oligonucléotidique étant éventuellement marquée de telle sorte qu'elle puisse être liée à un partenaire de liaison, ledit marqueur comprenant éventuellement de la biotine ;
comprenant en outre éventuellement une pluralité de billes comprenant une fraction configurée pour se lier au marqueur, la fraction comprenant éventuellement de la streptavidine, et la pluralité de billes comprenant éventuellement des billes magnétiques ; et/ou
comprenant au moins un tampon, ledit tampon présentant un pH d'environ 7,0, et/ou une concentration en ATP qui est supérieure à 1 mM, et est éventuellement d'environ 2 mM.

13. Article manufacturé comprenant au moins un récipient scellé, l'au moins un récipient scellé contenant au moins le mélange de deux protéines distinctes ou la protéine de fusion selon la revendication 9, l'article manufacturé comprenant en outre un matériau imprimé qui fournit une indication que le contenu de l'article manufacturé est destiné à être utilisé dans le séquençage d'ARN.

14. Article manufacturé selon la revendication 13, comprenant en outre un ou une combinaison de :
i) au moins une amorce oligonucléotidique destinée à être utilisée dans la synthèse d'ADNc, l'amorce oligonucléotidique comprenant un segment poly-T, et l'amorce oligonucléotidique étant éventuellement marquée de telle sorte qu'elle puisse être liée à un partenaire de liaison, ledit marqueur comprenant éventuellement de la biotine ;
ii) une pluralité de billes comprenant une fraction configurée pour se lier au marqueur, la fraction comprenant éventuellement de la streptavidine, et la pluralité de billes comprenant éventuellement des billes magnétiques.

15. Article manufacturé selon la revendication 14, comprenant en outre au moins un récipient scellé qui comprend un tampon ou des composants pour fabriquer le tampon, le tampon présentant un pH d'environ 7,0 et/ou une concentration en ATP qui est supérieure à 1 mM et est éventuellement une concentration d'environ 2 mM de l'ATP.
